(19) Europäisches Patentamt
European Patent Office
Office européen des brevets

(11) EP 3 895 710 A1

(12) # EUROPEAN PATENT APPLICATION

(43) Date of publication:
**20.10.2021 Bulletin 2021/42**

(51) Int Cl.:
***A61K 31/715*** *(2006.01)* ***A61K 31/732*** *(2006.01)*
***A61P 29/00*** *(2006.01)* ***A61P 35/00*** *(2006.01)*

(21) Application number: **20169672.1**

(22) Date of filing: **15.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **DSM IP Assets B.V.**
  **6411 TE Heerlen (NL)**
- **University Medical Center Groningen (UMCG)**
  **9713 GZ Groningen (NL)**
- **Wageningen University**
  **6708 WG Wageningen (NL)**

(72) Inventors:
- **BEUKEMA, Martin**
  **9713 GZ Groningen (NL)**
- **DE VOS, Paulus**
  **9713 GZ Groningen (NL)**
- **JERMENDI, Éva**
  **6708 WG Wageningen (NL)**
- **SCHOLS, Hendrik Arie**
  **6708 WG Wageningen (NL)**
- **VAN DEN BERG, Marco Alexander**
  **4303 Kaiseraugst (CH)**

(74) Representative: **Kurt, Manfred**
**DSM NUTRITIONAL PRODUCTS LTD.**
**Patent Department**
**Wurmisweg 576**
**4303 Kaiseraugst (CH)**

(54) **FOOD AND/OR FEED COMPOSITIONS TO MANAGE IMMUNE HOMEOSTASIS**

(57) This invention relates generally to compositions and methods for combating inflammatory disease and particularly to the use of food and/or feed compositions for preventing, reducing and/or treating inflammatory disorders, diseases, or discomforts, wherein the compositions comprising at least one specific polysaccharide.

EP 3 895 710 A1

## Description

The present invention relates to a polysaccharide that can be used to modulate immune responses. The invention further relates to the polysaccharide for use as a medicament. This invention relates generally to compositions and methods for improving general health and combating inflammatory diseases. The present invention also relates to an edible product or a pharmaceutical composition comprising the polysaccharide. The invention also relates to a method for preparation of an edible product or pharmaceutical composition comprising the polysaccharide. This invention relates particularly to the use of food and/or feed compositions for preventing, reducing and/or treating inflammatory disorders, diseases, or discomforts.

The immune system is a host defense system comprising many biological structures and processes within an organism that protects against disease. To function properly, an immune system must detect a wide variety pathogens, and distinguish them from the organism's own healthy tissues. In many species, there are two major subsystems of the immune system: the innate immune system and the adaptive immune system. Both subsystems use humoral immunity and cell-mediated immunity to perform their functions. Cell-mediated immunity is the activation of phagocytes, antigen-specific T-lymphocytes, and the release of various cytokines in response to antigens. Some of the main cellular elements of the cell-mediated immune system in providing protection against different pathogens are helper T cells (also known as T helper cells or CD4 cells) and cytotoxic T cells (also known as T cytotoxic cells or CD8 cells). Naive T cells, which are mature T cells that have yet to encounter an antigen, are converted into activated effector T cells (also known as T effector cells) after encountering antigen-presenting cells (so-called APCs). T cells can differentiate into different subsets: the specific T helper (Th) subset and T cytotoxic subset. The Th cells can also differentiate into specific T effector cells which are responsible for inducing specific pro-inflammatory responses against pathogens and helminths by secreting cytokines. Three types of Activated Effector T cells (so-called Teff cells or, in short, Teffs) can be distinguished, detecting antigens originating from various types:

1). Th1 cells, which primarily respond to intracellular pathogens and activate macrophages,
2). Th2 cells, which primarily respond to allergenic responses and stimulate B cells into producing antibodies,
3). Th17 cells, which primarily respond to extracellular pathogens.

T helper cells can also differentiate into T regulatory cells (so-called Treg cells or, in short, Tregs) which have anti-inflammatory properties and limit excessive pro-inflammatory responses of T effector cells. This regulation of responses by T regulatory cells is essential for intestinal homeostasis as an excessive pro-inflammatory response of T effector cells can lead to the development of inflammation, atopic disease and immunopathological diseases. However, an excessive T regulatory cell response is also not beneficial as it may suppress pro-inflammatory responses against pathogens excessively, leading to the development of infections. Therefore, there is a need for a method to obtain a balanced T cell composition managing immunity required to achieve intestinal homeostasis.

Th17 cells are a subpopulation of CD4+ T cells that are involved in the disease progression of many autoimmune and inflammatory disorders due to their secretion of pro-inflammatory IL-17 cytokines (Sandquist & Kolls, 2018, F1000Res.7, 205). Increased IL-17 levels have been detected in multiple autoimmune models in animals, as well as in human patients with various autoimmune disease syndromes, such as systemic lupus erythematosus, multiple sclerosis, inflammatory colitis, rheumatoid arthritis and psoriasis. In rheumatoid arthritis, Th17 cells may induce matrix metalloproteases and stimulate osteoclasts, which leads to the development of cartilage and bone destruction. IL-17 deficient mice or mice treated with an IL-17 receptor antagonist are resistant to the development of arthritis (Chen et al, 2011, Int Immunopharmacol. 11(5):536-542).

Regulatory T cells (Tregs) expressing Foxp3 (forkhead box P3 transcription factor) are essential for immunological tolerance, illustrated by uncontrolled effector T cell responses and autoimmunity upon loss of Foxp3 expression by Tregs, converting them into pro-inflammatory Th17 cells or pathogenic "exFox3p" cells (Yang et al, 2016, Mucosal Immunol. 9(2):444-457). Tregs can adopt specific effector phenotypes upon activation, reflecting the diversity of functional demands in the different tissues of the body. A particular lineage of Treg Foxp3+CD4+ cells are coexpressing the retinoic acid-related orphan receptor-$\gamma$t (ROR$\gamma$t), the master transcription factor for Th17 cells. Such Foxp3+ROR$\gamma$t+ Tregs have enhanced suppressive capacity in a T-cell transfer colitis model and were shown to have a regulatory function during the occurrence of autoimmune diabetes. Moreover, they can express the anti-inflammatory cytokine IL-10 and suppress the (uncontrolled and unwanted) proliferation of effector T cells, and thereby Tregs and specifically Foxp3+ROR$\gamma$t+ Tregs contribute to an optimal immune homeostasis during gut-specific immune responses. Loss of the balance between Th17 and Treg cells in favor of Th17 cells will break immune homeostasis in the host and lead to the development of autoimmune diseases.

Inflammatory (or also inflammation-related disorders), atopic and/or immunopathological diseases in human are for example diseases such as Alzheimer's, ankylosing spondylitis, arthritis (osteoarthritis, rheumatoid arthritis (RA), psoriatic arthritis), asthma, atherosclerosis, Crohn's disease, colitis, dermatitis, diverticulitis, fibromyalgia, hepatitis, irritable bowel syndrome (IBS), inflammatory bowel disease (IBD), systemic lupus erythematous (SLE), nephritism, Parkinson's disease, ulcerative colitis, auto-immune disease, asthma, atopy, cardio-vascular disease, diabetes, immune senescence, ischemia / reperfusion injury of the heart or of kidneys, feline infectious peritonitis, mastitis, psoriasis, sepsis, systemic lupus erymathosis, tumor metastasis, and visceral or cutaneous Leishmaniasis.

Most of these diseases are wide-spread. For example, arthritis is the most common cause of disability in the USA. More than 20 million individuals with arthritis have severe limitations in function on a daily basis. It can affect humans as well as animals. In the context of the present invention we use the term "patients" to describe the targeted individuals. These patients are susceptible to or suffering from inflammatory disease. Rheumatism or rheumatic disorder is a non-specific term for medical problems affecting the joints and/or connective tissue. The term "rheumatism" is still used in colloquial speech and historical contexts, but is no longer frequently used in medical or technical literature; there is no longer any recognized disorder simply called "rheumatism." The traditional term covers such a range of different problems that to ascribe symptoms to "rheumatism" is not to say very much. "Non-articular rheumatism," also known as "regional pain syndrome" or "soft tissue rheumatism," can cause significant discomfort and difficulty. Furthermore, arthritis and rheumatism between them cover at least 200 different conditions. Rheumatism and arthritis are general terms for acute and chronic conditions characterized by inflammation and pain. Rheumatism is a general category of conditions characterized by inflammation and pain in muscles and joints, including arthritis. Arthritis is characterized by inflammation of joints that causes swelling and pain. Types of arthritis include osteoarthritis, rheumatoid arthritis, ankylosing spondylitis (AS), and systemic lupus erythematosus (SLE). Rheumatic conditions include infectious arthritis, rheumatoid arthritis, arthritis due to rheumatic fever, arthritis due to trauma or degenerative joint disease, myositis, neurogenic arthropathy, bursitis, fibromyositis and hydroarthrosis. The cause of such diseases in not always fully understood but may be the result of other degenerative diseases, trauma, or auto-immune diseases such as SLE. Inflammation also occurs as a defensive response to host invasion by foreign agents and mechanical trauma that results in an immune response, e.g., microbial agents such as bacterial and viruses, toxins, alarm molecules, and neoplasia.

What these diseases and conditions, examples of inflammatory diseases, share in common is inflammation and the resulting pain. Prior methods for preventing and treating inflammatory diseases have generally focused on pain-killing and anti-inflammatory drugs. Typical methods have focused on oral medications such as steroidal cortisone derivatives and numerous non-steroidal anti-inflammatory drugs (NSAIDs). Unfortunately, these drugs almost always exhibit undesirable side effects. Other efforts have focused on joint implants such as the knee or hip implants. These methods are lengthy and complicated surgical procedures that force the patient to undergo costly invasive surgery and a significant recovery period requiring a rigorous and costly regimen of physical therapy. There is, therefore, a need for new methods for preventing and treating inflammatory diseases that avoids the undesirable side effects and costly surgical procedures characteristic of previous methods for preventing and treating inflammatory diseases.

Next to inflammation, several types of infections in humans (for example, upper respiratory tract infections like common colds and flu, vaginosis, meningitis, pneumonia, urinary tract infections, gastroenteritis, skin infection, cellulitis) are causing many days, weeks even years of discomfort in a human life. Leading to a lot of medical costs and lost working days, i.e. productivity. Classically, methods for preventing and treating infections are generally focused on antibiotics and drugs. Unfortunately, next to exhibiting undesirable side effects, excessive use of drugs and antibiotics leads to antimicrobial resistance (AMR), environmental pollution and unnecessary healthcare costs. There is, therefore, a need for new methods for preventing and treating infections that avoids the undesirable side effects and costs of previous methods for preventing and treating infections.

Another example in which the invention is applicable is in managing or preventing disease caused by unhealthy diets or the use of drugs. For example mucositis is an inflammation in the small intestine caused by chemotherapy such as the commonly applied doxorubicin. This drugs is causing mucosal damage and release of intracellular molecules such as DNA, RNA and heat shock proteins (also called alarm molecules or danger associated molecular patterns) that cause a severe inflammatory response with mucositis as a consequence. This can be prevented or reduced by this invention.

In animals gastrointestinal disturbances that involve inflammatory responses are widespread. In farm animals (pigs, poultry and ruminants) this phenomenon causes large economic losses. For example within the pig population total losses of all piglets born in the European Union amount to approximately 17% and a substantial proportion of these losses can be associated with infections via mucosal surfaces (Lallès et al., 2007, Proc Nutr Soc. 66(2):260-268). A transient anorexia in newly weaned pigs is considered a major cause of these problems that leads to gut dysfunction, increased sensitivity to enteric infections and diarrhea. Concomitant patho-physiological changes include 20-30% reduction in mucosal weight associated with villous atrophy (Lallès et al., 2004, Animal Research 53, 301-316), a compromised intestinal barrier function (Wijtten et al., 2011, Br J Nutr. 105(7):967-981), disturbances in the homeostasis of the gut microbiota (Bauer et al., 2006, Nutr Res Rev. 19(1):63-78) and in the anatomical and functional development of the immune system (Lallès et al., 2007, Proc Nutr Soc. 66(2):260-268). Also in poultry, enteric diseases significantly contribute to economic losses because of impaired animal performance, increased mortality, and reduced welfare of birds (Timbermont et al., 2011, Avian Pathol. 40(4):341-347). Nonspecific enteritis, coccidiosis, viral infections, dysbacteriosis and bacterial infections have been identified as major intestinal diseases that cause wet litter in poultry (Hermans et al., 2006, Vet Rec. 158(18):615-622). Although there are some species related differences, the underlying pathophysiological changes are quite similar to those in pigs. For instance, small intestinal development in newly hatched broilers deteriorates at low feed intake levels (Wijtten et al., 2012, Acta Agriculturae Scandinavica Section A-Animal Science, 62(1), 1 - 12).

In the past, in-feed antibiotic growth-promoting agents were used prophylactically to prevent these kind of problems. Nowadays, the prophylactic use of several antibiotics in farm animals is banned in the European Union or under increased pressure by the public opinion in other countries such as the United States due to concerns about antibiotic residues in food and increased antibiotic resistance in pathogens. Hence, also for animals there is a need for new methods for preventing and treating gastrointestinal disturbances that involve inflammatory responses.

What all these unwanted disorders, diseases, or discomforts have in common is a need for a healthy and balanced T cell composition managing immunity to achieve intestinal homeostasis which facilitates preventing, reducing and/or treating inflammatory disorders, diseases, or discomforts. More specifically, in treating autoimmune diseases and preventing, reducing and/or treating inflammatory disorders, diseases, or discomforts, it is important to enhance the suppressive function of Tregs or promote Treg proliferation in vivo. More preferably, in treating autoimmune diseases and preventing, reducing and/or treating inflammatory disorders, diseases, or discomforts, it is important to enhance the suppressive function of Foxp3+RORγt+ or Foxp3+RORγt+CD4+ Tregs or promote Foxp3+RORγt+ or Foxp3+RORγt+CD4+ Tregs proliferation in vivo.

Known methods in the art to stimulate Treg function or proliferation is through antigen stimulation, like immunomodulating cytokines, small molecular inhibitors or therapeutic antibodies.

US7651855 describes a method to suppress autoimmune responses involving the selection of CD4+ T cells, using anti-CD25 antibodies for isolation, Magnetic Activated Cell Sorting for purification, and subsequent ex vivo cultivation for enrichment of regulatory T cells and their use in immunotherapy.

Luu et al. (Clin. Transl. Immunology, 2017, 6(9):e56) discuss the contribution of bacterial and dietary components such as short-chain fatty acids (SCFAs), vitamin A-derived retinoic acid (RA) and dietary antigens to the development, functional maturation and maintenance of intestinal regulatory T cells (Treg cells), and their central role in the maintenance of intestinal homeostasis by restraining inappropriate immune responses in the healthy gut.

US20190358258 provide for a method of enhancing the proliferation of T regulatory cells (Treg cells) in vitro, by contacting Treg cells with one or more Treg stimulation agents, such as anti-CD3 or anti-CD28 antibodies, or anti-CD3/CD28-coated beads, or IL-2.

US20160193257 provides for pharmaceutical compositions containing human derived bacteria belonging to the genus Clostridium to induce proliferation or accumulation of regulatory T cells in the colon to suppress immune functions.

US20190307795 describes a method of metabolic engineering of T cells to express chimeric antigen receptors for treatment of inflammation at specific sites in the body.

Charbonnier et al. (J. Immunology, 2006, 177(6):3806-13) describe repetitive injections with immature dendritic cells (iDC) to trigger the induction of regulatory T cells to protect from collagen-induced arthritis.

US9433652 describes the use of bacteria from the genus Clostridium clusters IV and/or XIVa, or physiological active substrates derived thereof, to induce the accumulation of regulatory T cells in the colon to suppress or control T effector cells.

In spite of the disclosure of the prior art, there is a need for naturally occurring compounds or ingredients, that can be part of a consumable product that can be used to maintain and/or stimulate a balanced T cell composition managing immunity to achieve intestinal homeostasis which facilitates preventing, reducing and/or treating inflammatory disorders, diseases, or discomforts. The invention relates to a polysaccharide for use as a food or feed ingredient. The invention further relates to a polysaccharide for use as a nutritional supplement. The invention further relates to a polysaccharide for use as a medicament. This invention relates generally to compositions and methods for improving general health and combating inflammatory diseases. The present invention also relates to an edible product or a pharmaceutical composition comprising the polysaccharide. The invention also relates to a method for preparation of an edible product or pharmaceutical composition comprising the polysaccharide. This invention relates particularly to the use of food and/or feed compositions for preventing, reducing and/or treating inflammatory disorders, diseases, or discomforts.

Surprisingly, it was found that a specific polysaccharide (with regard to its pattern of esterification, a structural parameter for the distribution of esterified and/or non-esterified D-galacturonic acid residues in a polysaccharide) (or a mixture of specific polysaccharides) is useful in preventing, reducing and/or treating inflammatory disorders, diseases, or discomforts.

By "preventing, reducing and/or treating inflammatory disorders, diseases, or discomfort" it is mainly meant:

- minimizing the risk of contracting any of the above listed diseases, and/or

- ameliorating the related symptoms, and/or
- lessening pain or discomfort associated with any of the above listed diseases and/or
- lengthen the time between episodes where any of the above listed diseases can occur and/or
- improving human and animal overall health and welfare

The polysaccharide of the invention can be obtained from edible plants, being vegetables, fruits, herbs and spices. It can be produced commercially as a white to light brown powder. Natural sources for the polysaccharide of the invention can be: pears, apples, guavas, papayas, mango, avocado, quince, plums, gooseberries, bilberry, oranges, lemons, cherries, grapes, strawberries, potato, soy, sugar beet, chicory, carrot, tomato, olives, pea, parsley, celery, coriander, cumin, dill, fennel, lovage, angelica, anise, parsnip, rose hips, and (green) beans. This list is not exhaustive at all. Only the main sources are listed.

The polysaccharide of the invention is composed of a backbone of glycosyl building blocks up to 500 kDa in size. The molecular weight (MW) of the polysaccharide of the invention can vary between 0.370 and 500 kDa in size. Typically, when the polysaccharide is obtained from plants it is a mixture of molecules varying in size and the MW given is an average of the individual polysaccharides in the composition. In one embodiment, the average MW is minimally 100 kDa. In another embodiment, the average MW is below 100 kDa. In yet another embodiment, the MW is between 10 and 200 kDa. The invention also covers mixtures of polysaccharides from different origins and/or different MWs. Therefore, compositions of polysaccharides can be made covering the full spectrum of possible MWs between 0.370 and 500 kDa.

The polysaccharide may contain one or more alternating structural segments with a variant make-up. These variant structural segments include, but are not limited to, structural segments consisting of

(i) α-1,4-linked D-galacturonic acid units;

(ii) alternating α-(1,2)-linked L-rhamnosyl and α-1,4-linked D-galacturonosyl residues of which the former can be partially substituted at the O-4 and/or O-3 positions with one or more side chains such as α-(1,5)-L-arabinofuranose and/or β-(1,4)-D-galactopyranose. The oligosaccharide side chains may be linear or branched, and some of these side chains may be terminated with α-L-fucosides, L-rhamnose, L-arabinose, β-D-glucuronides, and 4-O-methyl beta-D-glucuronyl (as ferulic and coumaric acid) residues;

(iii) short stretches of α-1,4-linked D-galacturonic acid units partially substituted at the C-2 and/or C-3 positions with alternating glycosyl residues, including D-apiose, 3-C-carboxy-5-deoxy-L-xylose, 2-keto-3-deoxy-D-lyxo-heptulosaric acid, 2-keto-3-deoxy-D-manno-octulosonic acid, D-rhamnose, D-galactose, L-galactose, galacturonic acid, L-arabinose, xylose, 2-O-methyl xylose, 2-O-methyl fucose and L-aceric acid, linked together by at least 21 distinct glycosidic linkages;

(iv) sequences of α-1,4-linked D-galacturonic acid partially substituted at the C-2 and/or C-3 positions with apiose;

(v) sequences of α-1,4-linked D-galacturonic acid partially substituted at the C-2 and/or C-3 positions with D-xylose.

In all these segments, the D-galacturonic acid residues can be saturated or unsaturated. Typically the unsaturated bond is between positions C-4 and C-5.

The relative proportions of these different structural segments may vary significantly for different plant origins and the various derived commercial products. The relative proportions of these different structural segments in the polysaccharide can vary, depending on the source of the polysaccharide, on the extraction method and/or subsequent modifying or purification steps. The relative proportions of each different structural segment in the polysaccharide can vary between 0 and 100%, with the sum of all segments adding up to 100%. In a particular embodiment the relative proportion of the structural segment consisting of α-1,4-linked D-galacturonic acid units (i.e. segment i) either esterified and/or non-esterified (as schematically illustrated below) to the total polysaccharide of the invention is between 1% and 100%, more preferably between 10% and 100%, even more preferably between 20% and 100%, even more preferably between 50% and 100%. Other preferred ranges for the relative proportion of the structural segment consisting of α-1,4-linked D-galacturonic acid units either esterified and/or non-esterified (as schematically illustrated below) to the total polysaccharide of the invention are 50%-90%, 70%-100%, 60%-80% or 70%-95%.

In another particular embodiment the relative proportion of the structural segment consisting of alternating α-(1,2)-linked L-rhamnosyl and α-1,4-linked D-galacturonosyl residues of which the former can be partially substituted at the O-4 and/or O-3 positions with one or more side chains such as α-(1,5)-L-arabinofuranose and/or β-(1,4)-D-galactopyranose (i.e. segment ii) to the total polysaccharide of the invention is between 0% and 50%.

In another particular embodiment the relative proportion of structural segments iii, iv and v each vary between 0% and 25%. In another particular embodiment the relative proportion of structural segments iii, iv and v each is below 1%.

In another particular embodiment the ratio between structural segments i and ii in a particular polysaccharide of the invention varies between 0.1 and 10. In another particular embodiment the ratio between structural segments i and ii in a particular polysaccharide of the invention is below 5. In another particular embodiment the ratio between structural

segments i and ii in a particular polysaccharide of the invention is below 2. In another particular embodiment the ratio between structural segments i and ii in a particular polysaccharide of the invention is below 1.
The following illustration shows parts of the structure of the polysaccharide of the invention:

structural segment of the polysaccharide of the invention (non-methylesterified polygalacturonic acid)

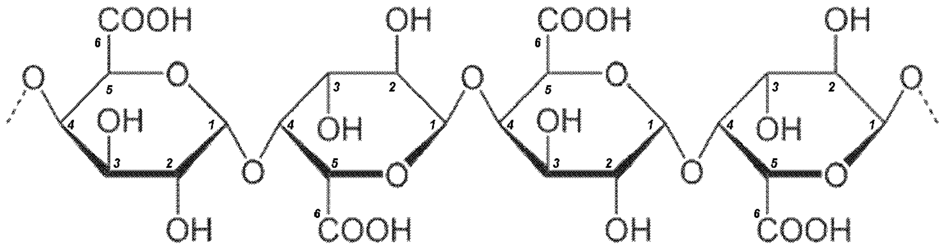

structural segment of the polysaccharide of the invention (methylesterified polygalacturonic acid)

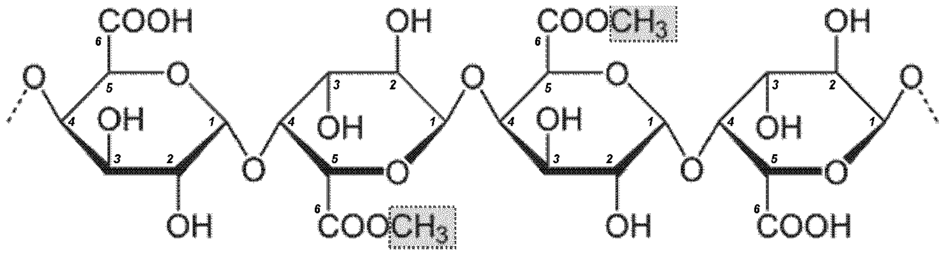

In the above shown structures the esterification is a methylesterification (indicated by the light gray box). The polysaccharide can be esterified and/or acylated with one group (such as a methyl group, $CH_3$, or an acetyl group, $COCH_3$) or with more than one group in the same oligomer structure. The polysaccharide can be esterified and/or acylated with one group (such as a methyl group, $CH_3$, or an acetyl group, $COCH_3$) or with more than one group on the same D-galacturonic acid residue. Acetylation usually occurs at the oxygen of the hydroxyl group on carbon position 2 and/or 3, while methylesterification usually occurs at the hydroxyl functionality of the carboxyl group at carbon position 6.

In the context of the invention the terms esterification, alkylation and acylation can be used interchangeable, and are used to indicate the derivatization of a hydroxyl group of the polysaccharide of the invention. The hydroxyl group can be for example the hydroxyl group of a carboxylgroup, or a primary, secondary or tertiary hydroxyl group. The main alkyl or acyl groups for the esterification of the polysaccharide of the invention are, but not limited to, methyl, acetyl and feruloyl. Esterification can occur on any of the glycosyl building blocks of the polysaccharide of the invention.
Methylesterified polysaccharide residues are indicated via different names such as, but not limited to, methylesterified D-galacturonic acid, methylgalacturonic acid, methylgalacturonate, or galacturonic acid methyl ester; however, with all is meant the same: a galacturonic acid residue of the polysaccharide with a methyl group.
Acetylated polysaccharide residues are indicated via different names such as, but not limited to, acetylated galacturonic acid, galacturonosylacetate, 3-O-acetylgalacturonic acid, or 2-O-acetylgalacturonic acid; however, with all is meant the same: a galacturonic acid residue of the polysaccharide with an acetyl group.
Also other residues of the polysaccharide of the invention can be esterified, like, but not limited to, the glucuronic, rhamnose, arabinose, arabinofuranose, galactopyranose residues of the polysaccharide of the invention.
The various structural segments (i, ii, iii, iv and/or v) of the polysaccharide can be esterified. The major types of esterification are: O-methyl, O-acetyl and O-feruloyl. Not excluding any other types of esterification. Most of the esterifications reside in the structural segment consisting of $\alpha$-1,4-linked D-galacturonic acid units (i.e. segment i). Not excluding esterification of any of the glycosyl residues (D-galacturonic acid and/or rhamnose) in any of the other structural segments (for example segment ii, segment iii, segment iv and/or segment v) of the polysaccharide of the invention. The D-galacturonic acid residues can be thus present with free carboxyl and hydroxyl groups or esterified at the carboxyl or one or more of the hydroxyl groups. Esterification can occur as mono-esterification, but also as double esterification of single D-galacturonic acid residues. Not excluding any other numbers of esterification. The esterification on a single residue can be through a single type of alkyl group (for example, methyl) or a single type of acyl group (for example, acetyl). Not excluding any mixed type esterification. Thus, D-galacturonic acid can be methylesterified (leading to 0 or 1 methyl groups at the oxygen of the hydroxyl functionality of the carboxyl group of the C-6 per D-galacturonic acid residue) or can be acetylated (leading to 0, 1 or 2 acetyl groups respectively at the oxygen of the hydroxyl group on the

C-2 and/or C-3 per D-galacturonic acid residue). Not excluding methylation at the oxygen of the hydroxyl group on the C-2 and/or C-3 of D-galacturonic acid residues.

The degree of esterification (DE) is by definition the amount of esters (in moles) present per 100 moles of total galacturonic acids (unsubstituted D-galacturonic acid residues and substituted D-galacturonic acid residues summed together). As most polysaccharides are essentially having esterifications of the methyl-ester type, the DE is often expressed as the degree of methylesterification (i.e. DM). In that case, the degree of esterification is by definition the amount of methyl-esters (in moles) present per 100 moles of total galacturonic acids (unsubstituted D-galacturonic acid residues and substituted D-galacturonic acid residues summed together). In the case that the esterification is of the acetyl type, the DE is often expressed as the degree of acetylation (i.e. DA). In that case, the degree of esterification is by definition the amount of acetyl-esters (in moles) present per 100 moles of total galacturonic acids (unsubstituted D-galacturonic acid residues and substituted D-galacturonic acid residues summed together). In the case of multiple types of esterification in a single pectin sample, the DE is often expressed split in to a degree of methylesterification (i.e. DM) and a degree of acetylation (i.e. DA). These are calculated as described above. Alternatively, the DE can be expressed as the degree of esterification, defined by the amount of galacturonic acid residues modified with one or more esterifications, either being of the methyl or the acetyl type, (in moles) present per 100 moles of total galacturonic acids (unsubstituted D-galacturonic acid residues and substituted D-galacturonic acid residues summed together). Alternatively, the DE can be expressed as the degree of esterification, defined by the total amount of esters -either being of the methyl or the acetyl type- (in moles) present per 100 moles of total galacturonic acids (unsubstituted D-galacturonic acid residues and substituted D-galacturonic acid residues summed together).

In a specific embodiment, in the context of the invention and throughout the invention all the uses of and specifications of DE can be replaced by the amount of any type of ester (methyl, acetyl and/or feruloyl, either separate or summed together) (in moles) present per 100 moles of total specified residue (being either galacturonic acid, glucuronic acid, rhamnose, arabinose, arabinofuranose or galactopyranose, either separate or summed together) of the polysaccharide. Similarly, the RDES and/or DEC specifications could be interpreted and calculated using any type of ester (being methyl, acetyl and/or feruloyl, either separate or summed together) and any type of residue (being galacturonic acid, glucuronic acid, rhamnose, arabinose, arabinofuranose or galactopyranose, either separate or summed together) of the polysaccharide.

In the context of this invention the term degree of esterification (DE) is used, and the percentages described are always based on the amount of D-galacturonic acid residues which are substituted through esterification (for example, methylesterification). A DE of 50 means that 50% of all possible D-galacturonic acid residues are esterified (for example, methylesterified).

The following patent application is related to the use of esterified polysaccharides, more specifically it is related to the use of esterified polysaccharides with a specific distribution pattern of esterification. More preferably the esterified polysaccharides with a specific distribution pattern of esterification are pectins.

The following distinction is made among the esterified polysaccharides of the invention:

- Low-esterified polysaccharides
- High-esterified polysaccharides

DE values for polysaccharides typically range from 0% to 95%. Low-esterified polysaccharides have a degree of esterification (DE) of less than 50%. This means that less than 50% of the residues are esterified. High-esterified polysaccharides have a DE of more than 50%. This means that more than 50% of the residues are esterified.

Typically, when the polysaccharides are obtained from plants it is a mixture of polysaccharide varying molecules and the DE given is an average of the individual polysaccharides in the composition. In one embodiment, the DE is minimally 0%. In another embodiment, the average DE is below 95%. In yet another embodiment, the DE is between 0 and 95. Other preferred ranges for the DE of the polysaccharide of the invention are 0%-65%, 5%-95%, 5%-65%, 0%-50%, 50%-95% and 10%-80%. The invention also covers mixtures of polysaccharides from different origins and/or different DE. Therefore, compositions of polysaccharides can be made covering the full spectrum of possible DE between 0% and 95%.

DE values of polysaccharides of the invention can be calculated for methyl esters only, and are than referred to as the DM values. In one embodiment, the DM is minimally 0%. In another embodiment, the average DM is below 95%. In yet another embodiment, the DM is between 0 and 95. Other preferred ranges for the DM of the polysaccharide of the invention are 0%-65%, 5%-95%, 5%-65%, 0%-50%, 50%-95% and 10%-80%. The invention also covers mixtures of polysaccharides from different origins and/or different DM. Therefore, compositions of polysaccharides can be made covering the full spectrum of possible DM between 0% and 95%. Alternatively, the DE value of polysaccharides of the invention can be calculated for any type of esters, being for example but not limited to methyl, acetyl or feruloyl esters. Also, the DE value of polysaccharides of the invention can be calculated as the total of all types of esters, being for example but not limited to methyl, acetyl and feruloyl esters. In the latter cases the DE value can be higher than 100%.

DE values of polysaccharides of the invention can be calculated for acetyl esters only, and are than referred to as the DA values. In one embodiment, the DA is minimally 0%. In another embodiment, the DA is over 100%. In another embodiment, the DA is below 95%. In yet another embodiment, the DA is between 0 and 95. Other preferred ranges for the DA of the polysaccharide of the invention are 0%-65%, 5%-95%, 5%-65%, 0%-50%, 50%-95% and 10%-80%. The invention also covers mixtures of polysaccharides from different origins and/or different DA. Therefore, compositions of polysaccharides can be made covering the full spectrum of possible DA between 0% and 95%.

However, besides the DE also the distribution of the esters over the backbone structure of the polysaccharide is an important characteristic of polysaccharides. The degree of blockiness of esterification (DBE) is a structural parameter for the distribution of esterified D-galacturonic acid residues in a polysaccharide of the invention. When comparing polysaccharides with similar DE, high DBE polysaccharides (also known as HBE) have a more blockwise distribution of esterified D-galacturonic acid residues over all D-galacturonic acid residues of the polysaccharide of the invention, compared to low DBE polysaccharides (also known as LBE), which have a more random distribution of esterified D-galacturonic acid residues over all D-galacturonic acid residues of the polysaccharide of the invention.

The following distinction is made among the distribution of esters in esterified polysaccharides of the invention:

- Polysaccharides with many and smaller blocks (or randomly distributed) of esterified D-galacturonic acid residues (LBE)
- Polysaccharides with less and larger blocks (or blockwise distribution) of esterified D-galacturonic acid residues (HBE)

DBE values for polysaccharides of the invention typically range from 10% to 100%. The actual degree of blockiness of esters is important in relation to the DE of the polysaccharide of the invention. Polysaccharides with a low DE and a low DBE have a low number of esterified D-galacturonic acid residues, which are randomly distributed over all D-galacturonic acid residues (i.e. LBE). Polysaccharides with a low DE and a high DBE have a low number of esterified D-galacturonic acid residues, which are distributed blockwise over all D-galacturonic acid residues (i.e. HBE). Polysaccharides with a high DE and a low DBE have a high number of esterified D-galacturonic acid residues, which are randomly distributed over all D-galacturonic acid residues (i.e. LBE). Polysaccharides with a high DE and a high DB have a high number of esterified D-galacturonic acid residues, which are distributed blockwise over all D-galacturonic acid residues (i.e. HBE). DBE values of polysaccharides of the invention can be calculated for methyl esters only, and are than referred to as the DBME values. Alternatively, the DBE value of polysaccharides of the invention can be calculated for any type of esters, being for example but not limited to methyl, acetyl or feruloyl esters. Also, the DBE value of polysaccharides of the invention can be calculated as the total of all types of esters, being for example but not limited to methyl, acetyl and feruloyl esters.

Besides the DE and DBE also the distribution of the non-esterified D-galacturonic acid residues over the backbone structure is a characteristic of polysaccharides. The degree of blockiness (DB) is a structural parameter for the distribution of non-esterified D-galacturonic acid residues in a polysaccharide of the invention. When comparing polysaccharides with similar DE, high DB polysaccharides (also known as HB) have a more blockwise distribution of non-esterified D-galacturonic acid residues over all D-galacturonic acid residues of the polysaccharide of the invention, compared to low DB polysaccharides (also known as LB), which have a more random distribution of non-esterified D-galacturonic acid residues over all D-galacturonic acid residues of the polysaccharide of the invention.

The following distinction is made among the distribution of non-esterified D-galacturonic acid residues in esterified polysaccharides of the invention:

- Polysaccharides with many and smaller blocks (or randomly distributed) of non-esterified D-galacturonic acid residues (LB)
- Polysaccharides with less and larger blocks (or blockwise distribution) of non-esterified D-galacturonic acid residues (HB)

DB values for polysaccharides typically range from 10% to 100%. The actual degree of blockiness is important in relation to the DE of the polysaccharide of the invention. Polysaccharides with a low DE and a low DB have a high number of non-esterified D-galacturonic acid residues , which are randomly distributed over all D-galacturonic acid residues (i.e. LB). Polysaccharides with a low DE and a high DB have a high number of non-esterified D-galacturonic acid residues , which are distributed blockwise over all D-galacturonic acid residues (i.e. HB). Polysaccharides with a high DE and a low DB have a low number of non-esterified D-galacturonic acid residues , which are randomly distributed over all D-galacturonic acid residues (i.e. LB). Polysaccharides with a high DE and a high DB have a low number of non-esterified D-galacturonic acid residues , which are distributed blockwise over all D-galacturonic acid residues (i.e. HB). DB values of polysaccharides of the invention can be calculated with respect to non-methylesterified D-galacturonic acid residues only. Alternatively, the DB value of polysaccharides of the invention can be calculated with respect to any

type of non-esterified D-galacturonic acid residues, being for example but not limited to non-methylesterified, non-acetylesterified or non-feruloylesterified. Also, the DB value of polysaccharides of the invention can be calculated as the total of all types of non-esterified D-galacturonic acid residues, being for example but not limited to non-methylesterified, non-acetylesterified and non-feruloylesterified D-galacturonic acid residues.

As mentioned above the polysaccharides of the invention are present in almost all higher plants. Several by-products of the food industries are used for their extraction, such as citrus peels (by-product of citrus juice production), apple pommace (by-product of apple juice manufacture), sugar beet (by-product of the beet-sugar industry) and in a minor extend potatoes fibers, sunflower heads (by-product of oil production) and onions (May, 1990, Carbohydr. Polymers, 12: 79-99). Atypical process to extract the polysaccharides of the invention from the pomace or peels is in hot diluted mineral acid at pH1-3 at 50-90 °C during 3-12 hours (Rolin, 2002, In: Pectins and their Manipulation; Seymour G. B., Knox J. P., Blackwell Publishing Ltd, 222-239). Dry citrus peels contain 20 to 30% of the polysaccharides of the invention on a dry matter basis, lower amounts are present in dried apple pomace (10 to 15%) (Christensen, 1986, Pectins. Food Hydrocolloids, 3, 205-230). By adding alcohol (usually isopropanol but methanol or ethanol are also used) the pectins are precipitated. Finally, the gelatinous mass is pressed, washed, dried and ground (May, 1990, Carbohydr. Polymers, 12: 79-99). Depending on the process conditions, polysaccharides of the invention with various DE, DBE and/or DB are obtained. The polysaccharide of the invention is also known as pectic polysaccharide or pectin.

Low-methylesterified (LM) polysaccharides can be obtained by de-esterification of high-methylesterified (HM) polysaccharides, mainly by controlling the acidity, the temperature and the time during extraction. To produce other types of polysaccharides of the invention, esters can be hydrolysed by the action of acid or alkali either before or during an extraction, as concentrated liquid or in the alcoholic slurry before separation and drying. When alkali is used, the reaction has to be performed at a low temperature and in aqueous solutions to avoid β-eliminative degradation of the polymers (Kravtchenko et al, , 1992, Carbohydrate Polymers, 19, 115-124). LM polysaccharides can also be extracted with aqueous chelating agents such as hexametaphosphate (e.g. from potato) (Voragen et al., 1995, In: Food polysaccharides and their applications; Stephen A. M., New York: Marcel Dekker Inc, 287-339). The use of the enzymes pectin methyl-esterase (PME) for the production of LM polysaccharides can be an alternative for the chemical extraction (Christensen, 1986, Pectins. Food Hydrocolloids, 3, 205-230). The conditions and time of the different reactions are varied leading to polysaccharides with a different DE, even as low as a DE of zero.

The polysaccharides of the invention can also be modified according to state-of-the-art methods. One example of modification can be re-esterification and/or de-esterification. Re-esterification of the polysaccharide is typically done in acidified absolute methanol. De-esterification of the polysaccharide is typically done with sodium hydroxide. The combined action of re-esterification and/or de-esterification can result in a polysaccharide with a different DE as compared to the DE of the polysaccharide upon extraction from the natural source.

The DE can be determined by commonly known methods. For example, the degree of esterification can be determined using several methods such as titration (Food Chemical Codex, 1981), IR spectrometry (Gnanasambandam & Proctor, 2000, Food Chemistry, 68, 327-332; Haas & Jager, 1986, Journal of Food Science, 51(4), 1087-1088 ; Reintjes et al, 1962, Journal of food sciences, 27, 441-445) and NMR spectrometry (Grasdalen et al, 1988, Carbohydrate Research, 184, 183-191). Other methods using HPLC (Chatjigakis et al., 1998, Carbohydrate Polymers, 37, 395-408; Levigne et al., 2002, Food Hydrocolloids, 16(6), 547-550; Voragen et al, 1986, Food Hydrocolloids, 1(1), 65-70) and GC-headspace (Huisman et al, 2004, Food Hydrocolloids, 18(4), 665-668; Walter et al, 1983, Journal of Food Science, 48(3), 1006-1007) analysing the methanol content after saponification of the polysaccharides have been developed. A capillary electrophoresis (CE) method has been developed to determine the DE of the polymers as such (Jiang et al, 2005, Food Chemistry, 91, 551-555; Jiang et al, 2001, Journal of Agricultural and Food Chemistry, 49, 5584-5588; Zhong et al, 1998, Carbohydrate Research, 308, 1-8; Zhong et al, 1997, Carbohydrate Polymers, 32(1), 27-32). An advantage of the CE method is that the D-galacturonic acid content of the samples is not required to calculate the DE whereas the D-galacturonic acid values have to be known prior to the DE calculation following GC headspace and HPLC methods. Further detailed descriptions of methods to determine the DE, DM and/or DA are described in Handbook of Food Analytical Chemistry: Water, Proteins, Enzjmes, Lipids, and Carbohydrates (2005, John Wiley & Sons, Inc, eds. Wrolstad, Acree, Decker, Penner, Reid, Schwartz, Shoemaker, Smith and Sporns, chapter E3, Cell Wall Polysaccharides).

Other examples of modification of the polysaccharides of the invention can be sugar modification or size modification. Such modifications can be implied by various state-of-the-art methods, including enzymatic (for example with pectin methyl esterase, endo-arabinanase, endo-galactanase, RG-hydrolase and/or exo-arabinase), physical (for example with ball milling,) and/or chemical (for example amidation, or alkaline and acidic hydrolytic size reduction).

The DBE can be determined by commonly known methods. For example as described by Ralet et al. (Biomacromolecules 2012, 13, 1615-1624). The combined action of re-esterification and/or de-esterification can result in a polysaccharide with a different DBE as compared to the DBE of the polysaccharide upon extraction from the natural source.

The DB can be determined by commonly known methods. The DB is typically calculated as the number of D-galacturonic acid residues present as non-methyl-esterified monomers, dimers and trimers of D-galacturonic acid released by endo-polygalacturonase relative to the total amount of non-methyl-esterified D-galacturonic acid residues present and expressed as a percentage. Optionally, the incubation with endo-polygalacturonase is complemented with pectin lyase. In other words, the DB is calculated form the amount of non-methyl-esterified monomers (1°), dimers ($2^0$) and trimers ($3^0$) of D-galacturonic acid residues produced after the polysaccharide has been incubated with endo-polygalacturonase, divided by the total amount of non-methyl-esterified D-galacturonic acid residues present in the polysaccharide. An alternative to DB is the absolute degree of blockiness (DBabs), which is is calculated from the amount of non-methyl-esterified monomers ($1^0$), dimers ($2^0$) and trimers ($3^0$) of D-galacturonic acid residues produced after the polysaccharide has been incubated with endo-polygalacturonase, divided by the total amount of D-galacturonic acid residues (thus the sum of non-methyl-esterified and methyl-esterified residues) present in the polysaccharide. Also, the DBabs values for polysaccharides typically range from 10% to 100%.

Due to the possible heterogeneity in the structure of the polysaccharides of the invention (e.g. any isolated polysaccharide can harness a combination of the above describes segments i, ii, iii, iv and/or v), some parts of the polysaccharide are less accessible, soluble and/or amenable for the described analyses methods. In that case, additional enzymes can be applied to liberate further oligomeric structures from the polysaccharides which can be applied to the standard analyses as described in the current invention or known as state-of-the-art, in order to better quantify the various structural characterization parameters. Examples of such further enzymatic treatments and subsequent analyses are described by Broxterman et al. (2017, Carbohydrate Polymers, 177:58-66). A non-exhaustive list of enzymes is: endo-polygalacturonase, pectin lyase, bacterial pectin methyl esterase, fungal pectin methyl esterase, endo-arabinanase, endo-galactanase, RG-hydrolase and/or exo-arabinase), and combinations thereof. This allows for a further determination of the specific esters, their amount and their distribution (for example for methyl, acetyl and feruloyl esters).

The amount of non-methyl-esterified monomers, dimers and trimers of D-galacturonic acid from the enzyme digested polysaccharides was determined by HPAEC-PAD and corrected with HILIC-ESI-IT-MS data. HILIC-ESI-IT-MS was used to calculate the ratio of non- methyl-esterified and methyl-esterified diners and trimers of D-galacturonic acid after enzyme digestion. The total amount of D-galacturonic acid residues is determined by determining the uronic acid content (UA) by an automatic analyser (Skalar Analytical B.V., Breda, The Netherlands) according to the colourimetric m-hydroxydiphenyl assay described by Blumenkrantz and Asboe-Hansen (1973, Anal Biochem 54: 484-489). Hydrolysed polysaccharides were analysed using 0.04% (w/w) m-hydroxydiphenyl in 0.5% NaOH and 96% (w/w) $H_2SO_4$ with 0.0125 M sodiumtetraborate as reagents. The colour reaction was analysed at 99°C. The UV detector measured at 530 nm. D-galacturonic acid standards of 12.5, 25, 50, 75, and 100 μg/mL were used to make the calibration curve.
The combined action of re-esterification and/or de-esterification can result in a polysaccharide with a different DB (or DBabs) as compared to the DB (or DBabs) of the polysaccharide upon extraction from the natural source.

Surprisingly, it was found that the use of polysaccharides with a distinctive pattern of esterification over the polysaccharide was able to prevent, reduce and/or treat inflammatory disorders, diseases, or discomforts.

This effective and distinctive esterification pattern of the polysaccharide of the invention can be expressed as the relative degree of esterification scattering (RDES). The relative degree of esterification scattering (RDES) is a structural parameter for the scattered distribution of esterified D-galacturonic acid residues in a polysaccharide of the invention relative to the DE. When comparing polysaccharides with similar DE, high RDES polysaccharides (also known as HRDES) have more scattering of isolated esterified D-galacturonic acid residues in regions with esterified D-galacturonic acid residues of the polysaccharide of the invention, compared to low RDES polysaccharides (also known as LRDES), which have less scattering of isolated esterified D-galacturonic acid residues in regions with esterified D-galacturonic acid residues of the polysaccharide of the invention.

The following distinction is made among the scattering of isolated esterified D-galacturonic acid residues in esterified polysaccharides of the invention:

- Polysaccharides with more scattering of isolated esterified D-galacturonic acid residues in regions with esterified D-galacturonic acid residues of the polysaccharide of the invention, relative to the DE (i.e. HRDES)
- Polysaccharides with less scattering of isolated esterified D-galacturonic acid residues in regions with esterified D-galacturonic acid residues of the polysaccharide of the invention, relative to the DE (i.e. LRDES)

RDES values for polysaccharides of the invention typically range from 0% to 10%. RDES values of polysaccharides of the invention can be calculated for methylesters only, and in that case are referred to as RDMES values. Alternatively, the RDES value of polysaccharides of the invention can be calculated for any type of esters, being for example but not limited to methyl, acetyl or feruloyl esters. Also, the RDES value of polysaccharides of the invention can be calculated as the total of all types of esters, being for example but not limited to methyl, acetyl and feruloyl esters. In a specific embodiment the RDES value of the polysaccharide of the invention is less than 1.7%. In another embodiment the RDES value of the polysaccharide of the invention is less than 1.6%, or less than 1.5%. The RDES value of the polysaccharide of the invention can be between 0%-1.7%, 0%-1.6%, 0%-1.5%, 0.1%-1.7%, 0.2%-1.7%, 0.3%-1.7%, 0.3%-1.6% or 0.3%-1.5%.

The RDES value can be determined based on commonly known methods. Exemplified for methylesterification but also applicable to other types of esters, the RDES value of a polysaccharide is calculated as the ratio between the molar percentage of esterified D-galacturonic acid oligomers with a single methylesterification released by the combined action of endo-polygalacturonase and pectin lyase and the molar percentage of all methylesterified D-galacturonic acid oligomers released by the combined action of endo-polygalacturonase and pectin lyase, relative to the DE of the polysaccharide and expressed as a percentage. The amount of esterified D-galacturonic acid oligomers from the enzyme digested polysaccharides is determined by HPAEC-PAD and corrected with HILIC-ESI-IT-MS data. HILIC-ESI-IT-MS is used to calculate molar concentration of each oligomer and expressed as the percentage relative to all released monomers and oligomers, esterified an non-esterified. The combined action of re-esterification and/or de-esterification can result in a polysaccharide with a different RDES as compared to the RDES of the polysaccharide upon extraction from the natural source.

All RDES values in the present patent application are measured as described above and exemplified in example 2.

Another method to express the effective and distinctive esterification pattern of the polysaccharide of the invention is the degree of esterification clustering (DEC). The degree of esterification clustering (DEC) is a structural parameter for the clustered distribution of esterified D-galacturonic acid residues in a polysaccharide of the invention. When comparing polysaccharides with similar DE, high DEC polysaccharides (also known as HDEC) have more clustering of esterified D-galacturonic acid residues amongst all esterified D-galacturonic acid regions of the polysaccharide of the invention, compared to low DEC polysaccharides (also known as LDEC), which have less clustering of esterified D-galacturonic acid residues amongst all esterified D-galacturonic acid regions of the polysaccharide of the invention.

The following distinction is made among the clustering of esterified D-galacturonic acid residues in esterified polysaccharides of the invention:

- Polysaccharides with more clustering of esterified D-galacturonic acid residues distributed over all esterified D-galacturonic acid regions of the polysaccharide of the invention (HDEC)
- Polysaccharides with less clustering of esterified D-galacturonic acid residues distributed over all esterified D-galacturonic acid regions of the polysaccharide of the invention (LDEC)

DEC values for polysaccharides of the invention typically range from 0% to 100%. DEC values of polysaccharides of the invention can be calculated for methylesters only, and in that case are referred to as DMEC values. Alternatively, the DEC value of polysaccharides of the invention can be calculated for any type of esters, being for example but not limited to methyl, acetyl or feruloyl esters. Also, the DEC value of polysaccharides of the invention can be calculated as the total of all types of esters, being for example but not limited to methyl, acetyl and feruloyl esters.

In a specific embodiment the DEC value of the polysaccharide of the invention is more than 0.4 %. In another embodiment the DEC value of the polysaccharide of the invention is more than 0.5%, or more 0.6%. The DEC value of the polysaccharide of the invention can be between 0.4%-100%, 0.5%-100%, 0.6%-100%, 0.4%-90%, 0.4%-80%, 0.4%-70%, 0.4%-60% or 0.4%-50%.

The DEC value can be determined based on commonly known methods. The DEC value of a polysaccharide is calculated as the ratio between the molar percentage of esterified oligomers with minimally five D-galacturonic acid residues (methyl)esterified released by the combined action of endo-polygalacturonase and pectin lyase and the molar percentage of all non-(methyl)esterified D-galacturonic acid mono- and oligomers released by the combined action of endo-polygalacturonase and pectin lyase, and expressed as a percentage. The amount of esterified and non-esterified D-galacturonic acid oligomers from the enzyme digested polysaccharides is determined by HPAEC-PAD and corrected with HILIC-ESI-IT-MS data. HILIC-ESI-IT-MS is used to calculate the molar concentration of each oligomer and expressed as the percentage relative to all released monomers and oligomers, esterified an non-esterified. The combined action of re-esterification and/or de-esterification can result in a polysaccharide with a different DEC as compared to the DEC of the polysaccharide upon extraction from the natural source.

All DEC values in the present patent application are measured as described above and exemplified in example 3.

In a specific embodiment, the polysaccharide of the invention has a RDES value of less than 1.7% and a DEC value of more than 0.4%. In another embodiment, the polysaccharide of the invention has a RDES value of less than 1.6% and a DEC value of more than 0.4%. In another embodiment, the polysaccharide of the invention has a RDES value of less than 1.5% and a DEC value of more than 0.4%. In another embodiment, the polysaccharide of the invention has a RDES value of less than 1.7% and a DEC value of more than 0.5%. In another embodiment, the polysaccharide of the invention has a RDES value of less than 1.6% and a DEC value of more than 0.5%. In another embodiment, the polysaccharide of the invention has a RDES value of less than 1.5% and a DEC value of more than 0.5%. In another embodiment, the polysaccharide of the invention has a RDES value of less than 1.7% and a DEC value of more than 0.6%. In another embodiment, the polysaccharide of the invention has a RDES value of less than 1.6% and a DEC value of more than 0.6%. In another embodiment, the polysaccharide of the invention has a RDES value of less than 1.5% and a DEC value of more than 0.6%.

Therefore, the present invention relates to the use (U) of esterified polysaccharides (or a mixture of esterified polysaccharides) to treating inflammatory diseases in humans and animals (i.e. patients or subjects), wherein the polysaccharides have a relative degree of esterification scattering (RDES) of less than 1.7% and/or a degree of esterification clustering (DEC) of more than 0.4%.

The relative degree of esterification scattering (RDES) preferably determined by the method as described in example 2 and the degree of esterification clustering (DEC) of the present invention is preferably determined by the method as described in example 3.

Furthermore, the present invention relates to a method (M) of preventing, reducing and/or treating inflammatory disorders, diseases, or discomforts (lessening inflammatory diseases) by administering to patients (or subjects) esterified polysaccharides (or a mixture of esterified polysaccharides), wherein the polysaccharides have a relative degree of esterification scattering (RDES) of less than 1.7% and/or a degree of esterification clustering (DEC) of more than 0.4%.

In the context of the present invention, the polysaccharides can be obtained from any known sources. A list of suitable sources is given above. By using one of the processes as described above, the polysaccharides with the correct RDES or DEC are obtained.

Preferably, the RDES of the polysaccharide is less than 1.7%, more preferably less than 1.6%, especially preferred less than 1.5%.

Preferably, the DEC of the polysaccharide is more than 0.4%, more preferably more than 0.5%, especially preferred more than 0.6%.

Therefore, the present invention also relates to the use ($U_1$), which is use (U), wherein the polysaccharide has a RDES of less than 1.7% and/or a DEC of more than 0.4%.

Therefore, the present invention also relates to the use ($U_1$'), which is use (U), wherein the polysaccharide has a RDES of less than 1.7% and/or a DEC of more than 0.4%.

Therefore, the present invention also relates to the use ($U_1$"), which is use (U), wherein the polysaccharide has a RDES of less than 1.7% and/or a DEC of more than 0.4%.

Therefore, the present invention also relates to a method ($M_1$), which is method (M), wherein the polysaccharide has a RDES of less than 1.7% and/or a DEC of more than 0.4%.

Therefore, the present invention also relates to a method ($M_1$'), which is method (M), wherein the polysaccharide has a RDES of less than 1.7% and/or a DEC of more than 0.4%.

Therefore, the present invention also relates to a method ($M_1$"), which is method (M), wherein the polysaccharide has a RDES of less than 1.7% and/or a DEC of more than 0.4%.

Usually, the polysaccharide has a RDES of maximally 1.7%, preferably of maximally 1.6%, more preferably of maximally 1.5%. Therefore, there is a range RDES values of 0-1.7%, 0-1.6%, 0-1.5%, 0.1-1.5%, 0.2-1.5%.

Usually, the polysaccharide has a DEC of at least 0.4%, preferably of at least 0.5%, more preferably of at least 0.6%. Therefore, there is a range of DEC values of 0.4-100%, 0.5-100%, 0.6-100%, 0.6-80%.

Therefore, the present invention also relates to a method ($M_1$'"), which is method (M), wherein the polysaccharide has a RDES of 0-1.7%, (preferably 0-1.6%, more preferably 0-1.5%, especially preferred 0.1-1.5%, most preferred 0.2-1.5%) and/or a DEC of more than 0.4% (preferably 0.4 - 100%, more preferably 0.5-100%, especially preferred 0.6-100%, most preferred 0.6-80%).

Therefore, the present invention also relates to the use ($U_1$'"), which is use (U), wherein the polysaccharide has a RDES of RDES of 0-1.7%, (preferably 0-1.6%, more preferably 0-1.5%, especially preferred 0.1-1.5%, most preferred 0.2-1.5%) and/or a DEC of more than 0.4% (preferably 0.4 -100%, more preferably 0.5-100%, especially preferred 0.6-100%, most preferred 0.6-80%).

Commercial polysaccharides can be a mixture of several populations: the distribution of the substituents can differ in an intramolecular level (within one single polysaccharide polymeric chain) or in an intermolecular level (within one single polysaccharide sample comprising multiple individual polysaccharide polymeric chains). This holds for all substituents, thus the sugars as well as the esterifications, and therefore both categories are meant with the word 'substituents' in the following. The substituents can be distributed completely at random. This random distribution can follow an even distribution pattern, when the substituents are regularly distributed over a single pectin polymeric chain, leading to a more homogenous polysaccharide polymeric chain. If all polysaccharide polymeric chains in a single polysaccharide sample are of the same homogenous type, also the sample can be called homogeneous.
However, a single homogenous pectin polymeric chain can be present in a composition with other homogenous polysaccharide polymeric chains but having a different intramolecular (but still homogeneous) distribution of the substituents. In this case, the polysaccharide sample should be considered heterogeneous.

Furthermore, it is also possible to modify the esterified polysaccharide according to the present invention. One of the possible modifications is amidation. Amidated pectin is a modified form of pectin. In that case some of the galacturonic acid is converted with ammonia to carboxylic acid amide. This is done according to well-known processes. The presence of an amide group is typically at the C-6 position of the amidated D-galacturonic acid residues. If the polysaccharide is amidated, this is often expressed as the degree of amidation (i.e. DAM). In that case, the degree of amidation

is by definition the amount of amides (in moles) present per 100 moles of total galacturonic acids (free D-galacturonic acid residues and substituted D-galacturonic acid residues summed together).
In one particular embodiment of the invention the DAM of the polysaccharide is below 2%. In one particular embodiment of the invention the DAM of the polysaccharide is below 1%. In one particular embodiment of the invention the polysaccharide is not amidated.

Preferably the esterification type of the polysaccharide is either methylesterification and/or acetylation, more preferably methylesterification.

Therefore, the present invention also relates to the use ($U_2$), which is use (U), ($U_1$) ($U_1$'), ($U_1$") or ($U_1$'"), wherein the esterification type of the polysaccharide is either methylesterification and/or acetylation

Therefore, the present invention also relates to the use ($U_2$'), which is use (U), ($U_1$) ($U_1$'), ($U_1$") or ($U_1$'"), wherein the esterification type of the polysaccharide is methylesterification.

Therefore ,the present invention also relates to a method ($M_2$), which is method (M), ($M_1$), ($M_1$'), ($M_1$") or ($M_1$'"), wherein the esterification type of the polysaccharide is either methylesterification and/or acetylation.

Therefore, the present invention also relates to a method ($M_2$), which is method (M), ($M_1$), ($M_1$'), ($M_1$") or ($M_1$'"), wherein the esterification type of the polysaccharide is methylesterification.

Methods to characterize the different components (for example, D-galacturonic acid content, neutral sugar content, degree of methyl-esterification, degree of acetylation, degree of amidation, distribution of the non-methyl-esterified D-galacturonic acid residues, distribution of the methyl-esterified D-galacturonic acid residues, molecular weight) of natural, modified as well as commercial polysaccharides are well described in the PhD thesis of Stéphanie Guillotin (Studies on the intra- and intermolecular distributions of substituents in commercial pectins. Wageningen University, The Netherlands, 2005. ISBN 90-8504-265-8).

As described above, the specific polysaccharides are used to combating inflammatory disease of humans or animals. Also it can be used to prevent or cure pro-inflammatory events such as occur during the use of cytostatics for cancer treatment, or other discomfort during the use of drugs that causes visceral damage.

The term "patient" or "subject" means a human or other animal likely to develop or suffering from inflammatory disease, including avian, bovine, canine, equine, galline, feline, hircine, lapine, murine, musteline, ovine, piscine, porcine and vulpine animals. Preferably, the patient is a human, bovine, canine, feline, galline, ovine, porcine or avian.

The specific polysaccharides according to the present invention are used - as disclosed above- to prevent, reduce and/or treat inflammatory disorders, diseases, or discomforts.. This result is obtained by the fact that the specific polysaccharides according to our invention are surprisingly able to balance the population of different T cells, and thereby maintaining immune homeostasis. It was shown that polysaccharides with a low RDES and/or a high DEC (especially with respect to methylesterification) show a surprising positive effect on immune homeostasis, i.e. preventing an uncontrolled and unwanted proliferation of Th17 cells. It was also shown that polysaccharides with a low RDES and/or a high DEC (especially with respect to methylesterification) show a surprising positive effect on immune homeostasis, i.e. activation and/or proliferation of T regulatory cells. It was also shown that polysaccharides with a low RDES and/or a high DEC (especially with respect to methylesterification) show a surprising positive effect on immune homeostasis, i.e. preventing an uncontrolled and unwanted proliferation of Th17 cells through the activation and/or proliferation of T regulatory cells.

The amount of polysaccharides, which is used can vary, depending on the patient. It must be an amount which shows a sufficient effect. The actual daily consumed amount can vary between 10 mg and 50 gram. Depending on its actual use, such as a medicament, a pharmaceutical product, a nutritional supplement, a dietary supplement, part of a beverage, as a food ingredient, as a feed ingredient, the daily consumed amount of the polysaccharide of the invention can be 10 mg, or 20 mg, or 30 mg or 50 mg, or 100 mg, or 200 mg, or 250 mg, or 500 mg, or 1 gram, or 2 gram, or 5 gram, or 20 gram or 50 gram, or any range between these individual amounts. Depending on its actual use, such as medicament, nutritional supplement, dietary supplement, food ingredient, feed ingredient, the daily consumed amount of the polysaccharide of the invention can consumed in one or more daily dosages, being either 1, or 2, or 3, or 4, or 5, or 6, or 7 or 8, or 9 or 10 daily dosages.

The present invention relates to a composition comprising

(a) at least one therapeutically inert carrier and
(b) at least one esterified polysaccharide, wherein the esterified polysaccharide has a relative degree of esterification scattering (RDES) of less than 1.7%, (preferably 0-1.6%, more preferably 0-1.5%, especially preferred 0.1-1.5%, most preferred 0.2-1.5%).

The present invention relates to a composition comprising

(a) at least one therapeutically inert carrier and
(b) at least one esterified polysaccharide, wherein the esterified polysaccharide has a degree of esterification clustering (DEC) of more than 0.4 (preferably 0.4 -100%, more preferably 0.5-100%, especially preferred 0.6-100%, most preferred 0.6-80%).

The present invention relates to a composition comprising

(a) at least one therapeutically inert carrier and
(b) at least one esterified polysaccharide, wherein the esterified polysaccharide has a degree of esterification clustering (DEC) of more than 0.4% (preferably 0.4 -100%, more preferably 0.5-100%, especially preferred 0.6-100%, most preferred 0.6-80%) and/or a relative degree of esterification scattering (RDES) of 0-1.7%, (preferably 0-1.6%, more preferably 0-1.5%, especially preferred 0.1-1.5%, most preferred 0.2-1.5%).

An therapeutically inert carrier can be any ingredient, which is used in the formulation which is intended to be formulated. Such ingredients are useful in the production of the food, feed, dietary supplement or pharmaceutical product, or it can be added for other reasons. Such components can be e.g. colorants, antioxidants, fillers, pH buffer, taste masking substances, auxiliary agents, etc.

The invention relates to a process of preparing a product selected from a medicament, a pharmaceutical product, a nutritional supplement, a dietary supplement, a beverage, a food product, a feed product, said process comprising addition of the polysaccharide according to the invention in a concentration of at least 0.1 % by weight of the dry matter (meaning the same as wt-% or % w/w) that is contained in the final product. The polysaccharide according to the invention is preferably added in a concentration of at least 0.2% by weight, more preferably 0.3%-95% by weight, most preferably 1%-80% by weight of the dry matter that is contained in the final product. In a particularly preferred embodiment, the polysaccharide according to the invention represents at least 20% by weight, more preferably at least 30% by weight, even more preferably 60% by weight, and most preferably at least 80% by weight of the total amount of polysaccharides present in the final product.

The product comprising the polysaccharide according to the invention in a concentration of at least 0.1 % by weight of the dry matter that is contained in the final product, can be in any form suitable to its final application, including but not limited to a powder, a jelly, a gummy, a chewable, a tablet, a caplet, a capsule, a solid, a semi-solid or a liquid.

In a preferred embodiment, the food product comprising the polysaccharide according to the invention in a concentration of at least 0.1 % by weight of the dry matter in the final product, is a dairy product (such as a yogurt, a milk, a cheese), a savory product (such as a soup, a sauce, a crisp), a baked product (such as a cereal, a cake, a bread, a cookie, a bar).

In a preferred embodiment the product comprising the polysaccharide according to the invention in a concentration of at least 0.1 % by weight of the dry matter, also comprises a vitamin. Such vitamin can be vitamin A, vitamin B1, vitamin B2, vitamin B3, vitamin B5, vitamin B6, vitamin B7, vitamin B9, vitamin B12, vitamin C, vitamin D, vitamin E, vitamin K or combinations thereof.

In a preferred embodiment the product comprising the polysaccharide according to the invention in a concentration of at least 0.1 % by weight of the dry matter, also comprises an antioxidant. Such antioxidant can be a flavonoid, a polyphenol, a phenolic acid, a phenolic ester, a carotenoid, a terpenoid, a vitamin or combinations thereof.

In a preferred embodiment the product comprising the polysaccharide according to the invention in a concentration of at least 0.1 % by weight of the dry matter, also comprises an colorant. Such colorant can be a carotenoid (E160, E161, E164), a chlorophyllin (E140, E141), an anthocyanins (E163), betanin (E162), annatto (E160b), caramel coloring (E150a-d), carmine (E120), elderberry juice (E163), lycopene (E160d), paprika (E160c), turmeric (E100) or combinations thereof.

In a preferred embodiment the product comprising the polysaccharide according to the invention in a concentration of at least 0.1 % by weight of the dry matter, also comprises a probiotic.

In a preferred embodiment the product comprising the polysaccharide according to the invention in a concentration of at least 0.1 % by weight of the dry matter, also comprises a prebiotic.

In a preferred embodiment the product comprising the polysaccharide according to the invention in a concentration of at least 0.1 % by weight of the dry matter, also comprises a flavor. Such flavor can be grape, raspberry, orange, lemon, blueberry, cherry, watermelon, strawberry, apple, kiwi, banana, pineapple, mango, papaya, cinnamon, tangerine, melon, peach, chocolate, mint, peppermint or combinations thereof.

In a preferred embodiment the product comprising the polysaccharide according to the invention in a concentration of at least 0.1 % by weight of the dry matter, also comprises a sweetener. Such sweetener can be sucrose, erythritol, fructose, glucose, maltose, lactose, corn syrup, xylitol, sorbitol, or other sugar alcohols, inulin, mogroside, stevia, rebaudioside A, rebaudioside M, rebaudioside D, rebaudioside C, miraculin, monetin, thaumatin, aspartame, neotame, saccharin, sucralose or combinations thereof.

In a preferred embodiment the product comprising the polysaccharide according to the invention in a concentration of at least 0.1 % by weight of the dry matter, also comprises an inert carrier.

In a preferred embodiment the product comprising the polysaccharide according to the invention in a concentration of at least 0.1 % by weight of the dry matter, also comprises one or more auxiliary ingredients, which are useful to formulate the polysaccharide. Such auxiliary ingredients can be a salt (organic or inorganic zinc, sodium, potassium or calcium salts such as e.g. such as calcium acetate, calcium benzoate, calcium carbonate, calcium chloride, calcium citrate, calcium sorbate, calcium sulfate, potassium acetate, potassium benzoate, potassium carbonate, potassium chloride, potassium citrate, potassium sorbate, potassium sulfate, sodium acetate, sodium benzoate, sodium carbonate, sodium chloride, sodium citrate, sodium sulfate, zinc acetate, zinc benzoate, zinc carbonate, zinc chloride, zinc citrate, zinc sorbate, zinc sulfate), starch, sugar or sugar derivative (such as e.g. maltodextrin, sucrose, dextrin, glucose, lactose, sorbitol), small organic molecules, flour, cellulose or minerals.

The polysaccharide, which is ingested by the patient can be in any form. It is possible to use the polysaccharide as such or in a mixture with other ingredients. When used in a mixture, the amount in this mixture is then depending on the other ingredients as well as the form of the mixture.

The ingredients which are used are usually chosen with regard to the use of the mixture. The ingredient can serve to improve the properties of the mixture or when the mixture is used to be formulated into a final composition to improve the final composition.

The ingredients can serve one or more purposes. It is clear that such ingredients must be food or feed grade (depending on its use).

The polysaccharide can also be part of a food or feed product, whereas the food or feed product can be in any commonly known and used form.

Furthermore, the polysaccharide of the invention can be included in encapsulated form, it can be used as a coating, or as a coacervate.

The amount of polysaccharide, which is used can vary (depending on the patient and/or inflammatory disease targeted). It depends on the body weight. Usually an amount between 0.01 and 5 g polysaccharide with a RDES of less than 1.7% and/or a DEC higher than 0.4% per Kg body weight and per day is desired.

The amount polysaccharide in a specific food or feed product usually varies dependent on the food or feed product. It is also dependent on how much a consumer/animals eat of this food or feed product. The amount in a food or feed product should be in such an amount that by a usual consumption of that food or feed product the necessary dosage of polysaccharide is consumed.

The following examples serve to illustrate the invention.

## EXAMPLES

### Example 1

### Degree of Esterification

The polysaccharides used were commercial available lemon pectins. To determine the amount of esters (in this case methylesters) in the polysaccharides, a gas chromatography (GC) method was used (Huisman et al., 2004, Food Hydrocolloids 18: 665-668). Five mg of each polysaccharide was saponified using 1 ml of 0.1M NaOH for 24 hours (1 hour at 4 °C, followed by 23 hours at room temperature). in GC head-space vials that were immediately sealed with a Teflon lined rubber septum. To the polysaccharide blank, 1 ml of ultra-pure water was added. Gas chromatography was run on a HS-GC equipped with a flame ionisation detector and an automatic injection system. Column: DB-WAX $30\times0.53\times1.0$. The following conditions were used: helium as carrier gas with a flow rate of 20 ml/min. Column temperature was set at 40 °C for 5 min and then programmed to 150 °C at a rate of 10 °C/min. The injector was set at 200 °C and the detector performed at 225 °C. The integration was achieved using ChromCard software. Samples were heated at 50 °C for 10 min in the head-space sampler. Two ml of the head-space volatiles was automatically injected in 10 s on the column. To calculate the concentration of released methoxyl groups, a calibration curve was made with various methanol concentrations in the range of 0-12 $\mu$mol methanol per ml. Different dilutions were made of the saponified polysaccharide to ensure the methanol signal felt in the calibration range.

To determine the amount of D-galacturonic acid residues, polysaccharides were first, pre-hydrolyzed by adding 0.45 ml 72% w/w $H_2SO_4$ to 10 mg polysaccharide in screw-capped glass tubes cooled on ice. The tubes were incubated for 1 hour at 30°C in a heating block. Subsequently, 4.95 ml MilliQ was added to hydrolyze the polysaccharides and they were incubated for 3 h in a heating block at 100°C. The uronic acid content (UA) was analysed by an automatic analyser (Skalar Analytical B.V., Breda, The Netherlands) according to the colourimetric m-hydroxydiphenyl assay described by Blumenkrantz and Asboe-Hansen (1973, Anal Biochem 54: 484-489) and Thibault and Robin (1975, Automatisation du dosage des acides uroniques par la méthode de carbazol. Application au cas de matières pectiques. Ann.Technol.Agric., 24 pp 99-110). Hydrolysed polysaccharides were analysed using 0.04% (w/w) m-hydroxydiphenyl in 0.5% NaOH and 96% (w/w) $H_2SO_4$ with 0.0125 M sodiumtetraborate as reagents. The colour reaction was analysed

at 99°C. The UV detector measured the color at 530 nm. D-Galacturonic acid standards of 12.5, 25, 50, 75, and 100 μg/mL were used to make the calibration curve.

The degree of esterification (DE, or here DM as only the methylesters were used for the calculation) was calculated using the following formula (I) :

$$\text{(I)} \qquad DE = \frac{\text{Methanol (mg/g)} / 32}{\text{D-Galacturonic acid (mg/g)} / 176} \times 100\%$$

Thus, the DE is calculated by dividing the molar concentration of methanol released after saponification of the polysaccharide (in mmol methanol per gram of polysaccharide) by the molar concentration of D-Galacturonic acid released after acid hydrolysis of the polysaccharide (in mmol D-Galacturonic acid per gram of polysaccharide), expressed as percentage. The DE values of the polysaccharides are summarized in table 1.

Table 1: Degree of Esterification

| Polysaccharide | DE |
|---|---|
| LP1 | 19% |
| LP2 | 18% |
| LP3 | 49% |
| LP4 | 43% |

## Example 2

### Relative Degree of Esterification Scattering

To determine the effective and distinctive esterification pattern of the polysaccharide of the invention, the relative degree of esterification scattering (RDES) was determined. As the relative degree of esterification scattering (RDES) is a structural parameter for the scattered distribution of isolated esterified D-galacturonic acid residues in a polysaccharide, an enzymatic method was developed to determine the composition of the polysaccharides of the invention in more detail. The enzymes used were pectin lyase (EC 4.2.2.10) and endo-polygalacturonase from Kluyveromyces fragilis.

All polysaccharides of example 1 were dissolved in 50 mM sodium acetate buffer pH 5.2 (at 5 mg/ml). The hydrolysis was performed at 40 °C by incubation of the polysaccharide solution with 0.06 units pectin lyase for 6 hours followed by the addition of 0.35 units endo-polygalacturonase and incubated for another 18 hours. Enzyme doses were sufficient to hydrolyse the polysaccharide backbone completely within 24 hrs. Inactivation of enzymes was performed at 100 °C for 10 min and the digests were centrifuged (20,000×g, 20 °C, 15 min). The supernatants obtained were analysed by high performance size exclusion chromatography (HPSEC), high performance anion exchange chromatography (HPAEC-PAD/UV) and Ultra-High Pressure Liquid Chromatography HILIC-ESI-IT-MSn.

High performance size exclusion chromatography (HPSEC)

Polysaccharides before and after enzymatic digestion were analysed using an Ultimate 3000 system (Dionex, Sunnyvale, CA, USA). A set of four TSK-Gel super AW columns (Tosoh Bioscience, Tokyo, Japan) was used in series: first guard column (6 mm ID × 40 mm) and the columns 4000, 3000 and 2000 (6 mm × 150 mm). The column temperature was set to 55 °C. Samples (10 μL, 2.5 mg/ml) were eluted with filtered 0.2 M NaNO3 at a flow rate of 0.6 ml/min. The elution was monitored by refractive index detection (Shodex RI 101; Showa Denko K.K., Tokyo, Japan). Pectin standards from 10 to 100 kDa were used to estimate the molecular weight distribution of the polysaccharides.

High performance anion exchange chromatography (HPAEC-PAD/UV)

The polysaccharide digests were analysed and subsequently quantified using an ICS5000 High Performance Anion Exchange Chromatography system with Pulsed Amperometric detection (ICS5000 ED) (Dionex Corporation, Sunnyvale, CA, USA) equipped with a CarboPac PA-1 column (250 mm × 2 mm i.d.) and a CarboPac PA guard column (25 mm × 2 mm i.d.) and UV detection at 235 and 250 nm (HPAEC-PAD/UV) (Dionex). The two mobile phases were (A) 0.1 M NaOH and (B) 1 M NaOAc in 0.1 M NaOH and the column temperature was 20 °C (Broxterman & Schols,

2018). GalA DP 1-3 (Sigma-Aldrich, Steinheim, Germany) were used as standards for quantification. Before the analysis pectin digests were diluted using ultra-pure water to 0.5 mg/ml. They were injected (10 μl) and eluted at a flow rate of 0.3 ml/min. The elution profile was as follows: 0-55 min, linear 20-65% B; 55.1-60 minutes column washing with 100% B; finally, column re-equilibration with 20% B from 60.1 to 75 min. To calculate the concentration of released D-Galacturonic acid oligomers, calibration curves were made for the monomer (DP1), dimer (DP2) and trimer (DP3) of D-Galacturonic acid using various concentrations in the range of 0-50 μg oligomer per ml.

Ultra-High Pressure Liquid Chromatography HILIC-ESI-IT-MSn

The enzymatic digests of the polysaccharides were analysed using UHPLC in combination with ESI-IT-MSn on a Hydrophilic Interaction Liquid Chromatography (HILIC) BEH amide column (1.7 μm, 2.1 x 150 mm). The samples were centrifuged (15000xg, 10 min, RT) and diluted (with 50% (v/v) acetonitrile containing 0.1% formic acid, to a final concentration of 1 mg/mL). The eluents used were (A1) 99:1% (v/v) water/acetonitrile (water/ACN); (B1) 100% ACN, both containing 0.1% formic acid with a flow rate of 400 μL/min. The following elution profile was used: 0-1 min, isocratic 80% B; 1-46 min, linear from 80% to 50% B; followed by column washing: 46-51 min, linear from 50% to 40% B and column re-equilibration; 51.1-60 minutes isocratic 80% B with a flow rate of 400 μL/min. Oven and tray temperature were kept at 40°C. Mass spectra were acquired over the scan range m/z 300-2000 in the negative mode. Heated Electrospray Ionisation Ion Trap ionised the separated oligomers in an LTQ Velos Pro Mass Spectrometry (UHPLC-ESI-IT-MSn) coupled to the UHPLC. Standards of monomer, dimer and trimer of D-galacturonic acid was used to determine a calibration line. The values for the trimer of D-galacturonic acid were used to calculate the concentrations of the longer oligomers.

The relative degree of esterification scattering (RDES) was calculated using the following formula's (II), (III) and (IV) :

$$\text{(II)} \qquad X_O = \frac{mC_O}{mC_P} \times 100\%$$

$$\text{(III)} \qquad M_O = \frac{\sum_{DP2^1\text{-}DP20^1} X_O}{\sum_{DP2^n\text{-}DP20^n} X_O} \times 100\%$$

$$\text{(IV)} \qquad RDES = \frac{M_O}{DE}$$

Herein is mCo the molar concentration (in mmol/g) of an individual monomer or oligomer released after the enzyme digest, and $mC_P$ the sum of the molar concentrations (in mmol/g) of all individual monomers and oligomers released after the enzyme digest, and Xo the percentage of each individual monomer or oligomer to the total of individual monomers and oligomers released after the enzyme digest. $M_O$ is ratio of the summed percentage of oligomers with a single (methyl)esterification [i.e. $DP2^1$, $DP3^1$, $DP4^1$, $DP5^1$, $DP6^1$, $DP7^1$, $DP8^1$, through to $DP20^1$, including both saturated as well as unsaturated D-galacturonic acids, wherein the '1' represents the single esterification of the respective oligomer] to the summed percentage of all (methyl)esterified oligomers [i.e. $DP2^n$ through to $DP20^n$, including both saturated as well as unsaturated D-galacturonic acids, wherein the 'n' represents any number of esterification of the respective oligomer]. Finally, RDES is the ratio of single (methyl)esterified oligomers versus all (methyl)esterified oligomers relative to the degree of (methyl)esterification.

Thus, the RDES value of a polysaccharide is calculated by dividing the molar percentage of esterified D-galacturonic acid oligomers with a single (methyl)esterification released after the combined action of endo-polygalacturonase and pectin lyase, by the molar percentage of all (methyl)esterified D-galacturonic acid oligomers released after the combined action of endo-polygalacturonase and pectin lyase, relative to the DE of the polysaccharide and expressed as a percentage. The RDES values of the polysaccharides of example 1 are summarized in table 2.

Table 2: Relative Degree of Esterification Scattering

| | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|
| LP2 | $X_O$ | | | | | | 4.351 | 2.201 | | | | | | | | | | | | | 0.338 | | | | | | | | | 6.89 |
| LP3 | $X_O$ | | | | | | 10.81 | 9.846 | | | | | | | | | | | | | 0.651 | | | | | | | | | 21.31 |
| LP4 | $X_O$ | | | | | | 14.91 | 5.349 | | | | | | | | | | | | | 0.816 | | | | | | | | | 21.07 |
| | | | | | | | | | | | | | | | | | | | | | | | | | | | | | | |
| | | | | | | | DP3$^1$ | DP4$^1$ | DP4$^2$ | DP5$^1$ | DP5$^2$ | DP5$^3$ | DP6$^2$ | DP6$^3$ | DP6$^4$ | DP7$^3$ | DP7$^4$ | DP7$^5$ | UDP2$^2$ | UDP3$^2$ | UDP4$^1$ | UDP4$^2$ | UDP4$^3$ | UDP5$^2$ | UDP5$^3$ | UDP5$^4$ | UDP6$^4$ | UDP6$^5$ | UDP7$^4$ | $\Sigma X_O$ (DP2$^n$-DP20$^n$) |
| LP1 | $X_O$ | | | | | | 2.912 | 1.328 | 3.026 | 0.487 | 1.439 | 0.643 | 0.205 | 0.682 | 0.619 | 0.293 | 0.105 | 0 | 0.704 | 1.204 | 0.257 | 0.715 | 0.309 | 0.459 | 0.781 | 0.159 | 0.254 | 0.51 | 0.376 | 17.47 |
| LP2 | $X_O$ | | | | | | 4.351 | 2.201 | 4.414 | 0.542 | 1.775 | 0.517 | 0.579 | 1.011 | 0.509 | 0.347 | 0.231 | 0 | 0.99 | 0.781 | 0.338 | 1.083 | 0.221 | 0.177 | 0.818 | 0.107 | 0.248 | 0.247 | 0.213 | 21.70 |
| LP3 | $X_O$ | | | | | | 10.81 | 9.846 | 6.851 | 0.913 | 8.567 | 2.613 | 2.704 | 3.934 | 1.806 | 0.595 | 0.97 | 0.578 | 2.511 | 4.52 | 0.651 | 2.542 | 2.387 | 1.52 | 3.859 | 0.6 | 0.769 | 1.442 | 0.976 | 71.96 |
| LP4 | $X_O$ | | | | | | 14.91 | 5.349 | 14.15 | 2.954 | 6.281 | 0.944 | 2.462 | 2.319 | 0.583 | 0.041 | 0.348 | 0.556 | 1.141 | 0.967 | 0.816 | 2.349 | 0.274 | 0.143 | 1.323 | 0.173 | 0.264 | 0.279 | 0.218 | 58.84 |
| | | | | | | | | | | | | | | | | | | | | | | | | | | | | | | |
| | | | | $\Sigma X_O$ (DP2$^1$-DP20$^1$) | | | $\Sigma X_O$ (DP2$^n$-DP20$^n$) | | | | $M_O$ | | DE | | RDES | | | | | | | | | | | | | | | |
| LP1 | | | | 4.50 | | | 17.47 | | | | 25.75 | | 19 | | 1.36 | | | | | | | | | | | | | | | |
| LP2 | | | | 6.89 | | | 21.70 | | | | 31.75 | | 18 | | 1.76 | | | | | | | | | | | | | | | |
| LP3 | | | | 21.31 | | | 71.96 | | | | 29.61 | | 49 | | 0.60 | | | | | | | | | | | | | | | |
| LP4 | | | | 21.07 | | | 58.84 | | | | 35.81 | | 43 | | 0.83 | | | | | | | | | | | | | | | |
| | | | | | | | | | | | | | | | | | | | | | | | | | | | | | | |

**Example 3**

**Degree of Esterification Clustering**

To determine the effective and distinctive esterification pattern of the polysaccharide of the invention, the degree of esterification clustering (DEC) was determined. As the degree of esterification clustering (DEC) is a structural parameter quantifying the clustering of esterified D-galacturonic acid residues in a polysaccharide of the invention, the enzymatic method of example 2 was used to determine the composition of the polysaccharides of the invention in more detail. The amount of esterified and non-esterified D-galacturonic acid mono- and oligomers (saturated and unsatured) from the enzyme digested polysaccharides is determined by HPAEC-PAD and corrected with HILIC-ESI-IT-MS data, as described in example 2.

The degree of esterification clustering (DEC) was calculated using formula (II) from example 2 and formula (V):

$$\text{(V)} \qquad \text{DEC} = \frac{\sum_{\text{DP5}^{5+}\text{-DP20}^{5+}} X_O}{\sum_{\text{DP1}^{0}\text{-DP20}^{0}} X_O} \times 100\%$$

Thus, the DEC value of a polysaccharide is calculated by dividing the summed molar percentage of esterified oligomers of with minimally five D-galacturonic acid residues (methyl)esterified [i.e. DP5$^{5+}$ through to DP20$^{5+}$, including both saturated as well as unsaturated D-galacturonic acids, wherein the '5+' represents 5 or more ester groups present in the respective oligomer] released after the combined action of endo-polygalacturonase and pectin lyase, by the summed molar percentage of all non-(methyl)esterified D-galacturonic acid mono- and oligomers [i.e. DP1$^0$ through to DP20$^0$, including both saturated as well as unsaturated D-galacturonic acids, wherein the '0' indicates no ester groups of the respective monomer or oligomer] released after the combined action of endo-polygalacturonase and pectin lyase, and expressed as a percentage. The DEC values of the polysaccharides of example 1 are summarized in table 3.

Table 3: Degree of Esterification Clustering

| Polysaccharide | | | oligomer | DP1$^{0}$ | DP2$^{0}$ | DP3$^{0}$ | DP3$^{1}$ | DP4$^{1}$ | DP4$^{2}$ | DP5$^{1}$ | DP5$^{2}$ | DP5$^{3}$ | DP6$^{2}$ | DP6$^{3}$ | DP6$^{4}$ | DP7$^{3}$ | DP7$^{4}$ | DP7$^{5}$ | UDP2$^{2}$ | UDP3$^{2}$ | UDP4$^{1}$ | UDP4$^{2}$ | UDP4$^{3}$ | UDP5$^{2}$ | UDP5$^{3}$ | UDP5$^{4}$ | UDP6$^{4}$ | UDP6$^{5}$ | UDP7$^{4}$ | |
|---|---|---|---|---|---|---|---|---|---|---|---|---|---|---|---|---|---|---|---|---|---|---|---|---|---|---|---|---|---|---|
| | | | MW | 194 | 370 | 546 | 560 | 736 | 750 | 912 | 926 | 940 | 1102 | 1116 | 1130 | 1292 | 1306 | 1320 | 380 | 556 | 718 | 732 | 746 | 908 | 922 | 936 | 1112 | 1126 | 1288 | |
| LP1 | amount | mg/g | | 52 | 74 | 290 | 20 | 12 | 27 | 5 | 16 | 7 | 3 | 9 | 8 | 5 | 2 | 0 | 3 | 8 | 2 | 6 | 3 | 5 | 9 | 2 | 3 | 7 | 6 | |
| LP2 | amount | mg/g | | 49 | 77 | 275 | 30 | 20 | 41 | 6 | 20 | 6 | 8 | 14 | 7 | 6 | 4 | 0 | 5 | 5 | 3 | 10 | 2 | 2 | 9 | 1 | 3 | 3 | 3 | |
| LP3 | amount | mg/g | | 12 | 39 | 62 | 61 | 73 | 52 | 8 | 80 | 25 | 30 | 44 | 21 | 8 | 13 | 8 | 10 | 25 | 5 | 19 | 18 | 14 | 36 | 6 | 9 | 16 | 13 | |
| LP4 | amount | mg/g | | 19 | 67 | 123 | 102 | 48 | 130 | 33 | 71 | 11 | 33 | 32 | 8 | 1 | 6 | 9 | 5 | 7 | 7 | 21 | 3 | 2 | 15 | 2 | 4 | 4 | 3 | |
| | | | | | | | | | | | | | | | | | | | | | | | | | | | | | | $mC_e$ |
| LP1 | $mC_O$ | mmol/g | | 0.268 | 0.2 | 0.53 | 0.035 | 0.016 | 0.037 | 0.006 | 0.017 | 0.008 | 0.002 | 0.008 | 0.007 | 0.004 | 0.001 | 0 | 0.009 | 0.015 | 0.003 | 0.009 | 0.004 | 0.006 | 0.009 | 0.002 | 0.003 | 0.006 | 0.005 | 1.21 |
| LP2 | $mC_O$ | mmol/g | | 0.253 | 0.209 | 0.503 | 0.054 | 0.027 | 0.054 | 0.007 | 0.022 | 0.006 | 0.007 | 0.012 | 0.006 | 0.004 | 0.003 | 0 | 0.012 | 0.01 | 0.004 | 0.013 | 0.003 | 0.002 | 0.01 | 0.001 | 0.003 | 0.003 | 0.003 | 1.233 |
| LP3 | $mC_O$ | mmol/g | | 0.064 | 0.106 | 0.113 | 0.109 | 0.099 | 0.069 | 0.009 | 0.087 | 0.026 | 0.027 | 0.04 | 0.018 | 0.006 | 0.01 | 0.006 | 0.025 | 0.046 | 0.007 | 0.026 | 0.024 | 0.015 | 0.039 | 0.006 | 0.008 | 0.015 | 0.01 | 1.01 |
| LP4 | $mC_O$ | mmol/g | | 0.097 | 0.182 | 0.226 | 0.183 | 0.066 | 0.174 | 0.036 | 0.077 | 0.012 | 0.03 | 0.028 | 0.007 | 0.001 | 0.004 | 0.007 | 0.014 | 0.012 | 0.01 | 0.029 | 0.003 | 0.002 | 0.016 | 0.002 | 0.003 | 0.003 | 0.003 | 1.227 |
| | | | | | | | | | | | | | | | | | | DP7$^{5}$ | | | | | | | | | | UDP6$^{5}$ | | ΣX$_O$ (DP5$^{5+}$-DP20$^{5+}$) |
| LP1 | $X_O$ | | | | | | | | | | | | | | | | | 0 | | | | | | | | | | 0.51 | | 0.51 |
| LP2 | $X_O$ | | | | | | | | | | | | | | | | | 0 | | | | | | | | | | 0.247 | | 0.25 |
| LP3 | $X_O$ | | | | | | | | | | | | | | | | | 0.578 | | | | | | | | | | 1.442 | | 2.02 |
| LP4 | $X_O$ | | | | | | | | | | | | | | | | | 0.556 | | | | | | | | | | 0.279 | | 0.84 |
| | | | | DP1$^{0}$ | DP2$^{0}$ | DP3$^{0}$ | | | | | | | | | | | | | | | | | | | | | | | | ΣX$_O$ (DP1$^{0}$-DP20$^{0}$) |
| LP1 | $X_O$ | | | 22.18 | 16.53 | 43.83 | | | | | | | | | | | | | | | | | | | | | | | | 82.53 |
| LP2 | $X_O$ | | | 20.56 | 16.94 | 40.8 | | | | | | | | | | | | | | | | | | | | | | | | 78.3 |
| LP3 | $X_O$ | | | 6.323 | 10.49 | 11.22 | | | | | | | | | | | | | | | | | | | | | | | | 28.04 |
| LP4 | $X_O$ | | | 7.935 | 14.8 | 18.43 | | | | | | | | | | | | | | | | | | | | | | | | 41.16 |
| | | | | ΣX$_O$ (DP5$^{5+}$-DP20$^{5+}$) | | | ΣX$_O$ (DP1$^{0}$-DP20$^{0}$) | | | | | | | | DEC | | | | | | | | | | | | | | | |
| LP1 | | | | 0.51 | | | 82.53 | | | | | | | | 0.618 | | | | | | | | | | | | | | | |
| LP2 | | | | 0.25 | | | 78.3 | | | | | | | | 0.315 | | | | | | | | | | | | | | | |
| LP3 | | | | 2.02 | | | 28.04 | | | | | | | | 7.206 | | | | | | | | | | | | | | | |
| LP4 | | | | 0.84 | | | 41.16 | | | | | | | | 2.029 | | | | | | | | | | | | | | | |

## Example 4

### Mice trial

C57BL6 female mice (10 weeks old) were obtained from Janvier Laboratories, France. All mice were acclimatized for 1.5 week prior to the start of the experiment. Animals were co-housed with a total number of 4 mice in individual ventilated cages. Mice were fed ad libitum with standard rodent chow diet (RHB-B; AB Diets, Woerden, The Netherlands). The ingredients of the diet specified by supplier were as follows: wheat, meat meal (80% sterilized), yellow dent corn, whole oats, wheat middlings, alfalfa, soya oil, dried yeast, dicalcium phosphate, calcium carbonate, NaCl, DL-methionine, vitamins, and trace elements. Mice were supplied with sterilized drinking water from the tap and water bottles were changed once a week.

Polysaccharides of example 1 were administered twice a day through oral gavage in a volume of 250 ul per gavage (6 mg/ml). Control mice received 250 ul of sterile water only. After 7, 14, 21 or 28 days of Polysaccharide administration, mice were sacrificed by cervical dyslocation. Digesta from duodenum, jejunum, ileum, caecum, proximal colon and distal colon were separately collected for microbiota analysis and determination of fermentation profiles. The spleen was collected as representative of systemic immunity and mesenteric lymph nodes was also collected as representative of intestinal immunity.

## Example 5

### T cell frequencies healthy mice

To study the effect of the polysaccharides of example 1 on immune status, T cells were isolated and quantified with flow cytometry. To this end, cells were isolated from the mesenteric lymph nodes for immune cell staining according to methods described before (Elderman et al., 2018, Biol Sex Differ. 9:26). Mesenteric lymph node cells were mechanically disrupted between two microscopy slides in 3 ml ice-cold Roswell Park Memorial Institute 1640 medium (RPMI) containing 10% (v/v) heat-inactivated fetal calf serum (FCS). Falcon tubes with cell strainer caps (Corning, Amsterdam, the Netherlands) (35 $\mu$m) were used to remove cell clumps before the cells were counted and used for staining.

All antibodies were diluted and supplemented to a volume of 25 $\mu$l with FACS™ (Fluorescence-Activated Cell Sorting) buffer [Phosphate-Buffered Saline (PBS) + 10% FCS (v/v)]. Approximately 1 $\times$ $10^6$ spleen cells were incubated in Zombie NIR (for dead/live staining) for 30 min. After washing with FACS buffer (2 times), the supernatant was discarded and the cells were incubated for 20 minutes in FACS buffer [PBS+10% FCS (v/v)] containing 20% (v/v) normal rat serum (Jackson, Newmarket, UK) on ice and in the dark, to prevent non-specific antibody binding. This was followed by incubation in an extracellular antibody mix for 30 minutes on ice and in the dark (Table 4). After 2 washing steps, the cells were fixed in BD FACS™ Lysing Solution (BD Biosciences, Breda, the Netherlands) for 30 minutes on ice and in the dark. Then cells were washed twice with 1x eBioscience™ PPermeabilization Buffer (ThermoFisher Scientific, Vienna, Austria) after which they were incubated with an intracellular blocking medium [20% (v/v) normal rat serum (Jackson, Cambridgeshire, United Kingdom) in permeabilization buffer] for 20 min. After washing, the cells were incubated with the intracellular antibody mix for 30 minutes on ice and in the dark (Table 4). This was followed by 2 washing steps with permeabilization buffer. Finally, the cells were taken up in 200 $\mu$l ice-cold 2%(v/v) FACS buffer and stored at 4°C until

analysis within 24 hrs. Fluorescence Minus One (FMO) controls were used to set the gates.

Table 4: antibodies and staining solutions

| Marker | T cell subset | Dilution | Company (catalogue number) | Clone |
|---|---|---|---|---|
| CD4-PE-Cy7 | CD4 | 100x | Biolegend (100422) | GK1.5 |
| CD3 - bv605 | CD3 | 25x | Biolegend (100237) | 17A2 |
| CD8-PerCP-Cy5.5 | CD8 | 50x | Biolegend (100734) | 53-6.7 |
| Tbet-bv421 | Th1 | 10x | Biolegend (644816) | 4B10 |
| Gata3-AF647 | Th2 | 100x | BD (560068) | L50-823 |
| RORγt-PE | Th17 | 100x | eBioscience (12-6981-80) | B2D |
| FoxP3-Fitc | Treg | 50x | eBioscience (11-5773-82) | FJK-16JS |
| Zombie NIR | dead/live | 1000x | Biolegend (423105) | n.a. |

Flow cytometry of T cells

Samples were analyzed with a FACSVerse flow cytometer system (BD Biosciences Franklin Lakes, USA), using the FACSsuite software. Analysis was performed by FlowJo version 10 software (FlowJo, LLC, Oregon, USA). The different probes on the antibodies (Table 4) are used to distinguish the different cell types. In table 5, the presence of the different T cell populations are given for Th1, Th2, Th17 and Treg as percentages of CD4+ cells. The presence of Foxp3+Rorγt+ Tregs are given as percentage of the Foxp3+CD4+ positive cells.

Table 5: T cell frequencies

| | Control | | | | LP1 | | | | LP2 | | | | LP3 | | | | LP4 | | | |
|---|---|---|---|---|---|---|---|---|---|---|---|---|---|---|---|---|---|---|---|---|
| RDES | n.a. | | | | 1.36% | | | | 1.76% | | | | 0.60% | | | | 0.83% | | | |
| DEC | n.a. | | | | 0.618% | | | | 0.315% | | | | 7.206% | | | | 2.029% | | | |
| | | | | | | | | | | | | | | | | | | | | |
| | week 1 | | week 4 | | week 1 | | week 4 | | week 1 | | week 4 | | week 1 | | week 4 | | week 1 | | week 4 | |
| T cell | average | st.dev. | average | st.dev. | average | st.dev. | average | st.dev. | average | st.dev. | average | st.dev. | average | st.dev. | average | st.dev. | average | st.dev. | average | st.dev. |
| Th1 | 0.79 | 0.18 | 1.24 | 0.35 | 1.55 | 0.36 | 0.84 | 0.17 | 0.79 | 0.46 | 1.48 | 0.48 | 1.27 | 0.41 | 0.95 | 0.29 | 1.99 | 0.70 | 1.42 | 0.51 |
| Th2 | 1.11 | 0.29 | 1.25 | 0.16 | 2.38 | 1.03 | 1.36 | 0.19 | 1.51 | 0.47 | 1.11 | 0.18 | 2.20 | 0.85 | 1.37 | 0.19 | 3.17 | 1.50 | 2.44 | 1.15 |
| Th17 | 1.39 | 0.44 | 1.46 | 0.19 | 1.48 | 0.47 | 1.30 | 0.38 | **2.36** | 0.66 | **2.24** | 0.77 | 1.63 | 0.80 | 1.48 | 0.47 | 2.08 | 0.99 | 1.52 | 0.46 |
| Fox3p+ Treg | 16.49 | 1.44 | 15.66 | 2.55 | 13.97 | 1.90 | 13.80 | 0.96 | 13.80 | 2.63 | 18.39 | 2.79 | 14.09 | 1.24 | 13.32 | 0.99 | 14.21 | 2.37 | 13.06 | 1.26 |
| Foxp3+ RORγt+ Treg | 6.54 | 1.19 | 7.37 | 1.38 | 9.06 | 2.19 | 10.04 | 1.43 | 8.18 | 1.20 | 9.48 | 1.01 | 7.56 | 0.93 | 11.68 | 2.09 | 10.06 | 3.54 | 11.73 | 1.67 |

Surprisingly, mice fed with diets supplemented with polysaccharides with a relative degree of esterification scattering (RDES) of less than 1.7% and/or a degree of esterification clustering (DEC) of more than 0.4% showed a significant increase in the number of Foxp3+RORyt+ Treg cells (indicated by black cells), suggesting a well-balanced immune status. While mice fed with diets supplemented with polysaccharides with a RDES of more than 1.7% and/or a DEC of less than 0.4% showed a significant but unwanted increase in Th17 cells (indicated by grey cells), suggesting an unbalanced T cell population, indicative of an inflammation status.

These results suggest that the use esterified polysaccharides with relative degree of esterification scattering (RDES) of less than 1.7% and/or a degree of esterification clustering (DEC) of more than 0.4% can be beneficial for treating, preventing, and/or reducing immune-mediated diseases and inflammatory diseases in mammals.

**Example 6**

**Doxorubicin induced mucositis**

To evaluate if the esterified polysaccharides with relative degree of esterification scattering (RDES) of less than 1.7% and/or a degree of esterification clustering (DEC) of more than 0.4% have a preventive and/or reducing effect on inflammatory diseases, ileitis (mucositis in the ileum) was induced in mice by administration of doxorubicin (Sigma, St.

Louis, MO, USA). A similar set-up for the mice trial was used as described in example 5.

Doxorubicin was dissolved in sterile 0.9% sodium chloride and stored in aliquots at 4°C. Pectin was dissolved in sterile water and administered by gavage to mice for 10 days, twice a day at a dose of 1.5 mg per gavage. On day 8, doxorubicin was injected intraperitoneally (IP) at 10 mg/kg. Mice were sacrificed on day 10 (48 h after doxorubicin). Animals receiving water or pectins only by gavage served as controls (see table 6). After the collection of tissue samples, mice were sacrificed by cervical dislocation.

Table 6: Experimental set-up induced mucositis trial

| Experimental group | Oral gavage | Intraperitoneal injection |
|---|---|---|
| Control | Water | Physiological salt |
| Doxorubicin-induced mucositis | Water | Doxorubicin |
| Control LP1 | LP1 | Physiological salt |
| Control LP3 | LP3 | Physiological salt |
| Control LP4 | LP4 | Physiological salt |
| Mucositis + LP1 | LP1 | Doxorubicin |
| Mucositis + LP3 | LP3 | Doxorubicin |
| Mucositis + LP4 | LP4 | Doxorubicin |

Histology

Ileum samples were fixed in 4% paraformaldehyde in PBS and embedded in paraffin. Paraffin sections of 4 μm were cut and Hematoxylin & Eosin (H&E) staining was performed on the slides. Villi length and thickness (see table 7) were determined from 10 consecutive villi structures of 3 different ileum segments.

Neutrophil Counts in Peritoneal Lavage

Peritoneal lavage was collected by injection and aspiration of 2 ml PBS. The total number of living cells in the peritoneal lavage, lysed with lyserglobin, was counted using a Z™ Series coulter counter® (Beckman Coulter, Brea, CA, USA). The lavage was diluted to 500,000 cells/ml, and 100 μl of cell solution was applied for cytospin preparation. The cytospin slides were stained with Giemsa (Merck Millipore, Billerica, MA, USA) for 1 hour at room temperature. The stained slides were scanned using a Hamamatsu slide scanner (Hamamatsu Photonics, Hamamatsu, Japan), and neutrophils were counted using morphological features in 250 cells. The total number of neutrophils (see table 8) was calculated using the total cell count of the peritoneum and the neutrophil counts from cytospin preparations.

Table 7: Villi length and thickness

| | Control | | Doxorubicin | | Doxorubicin + LP1 | | Doxorubicin + LP3 | | Doxorubicin + LP4 | |
|---|---|---|---|---|---|---|---|---|---|---|
| RDES | n.a. | | n.a. | | 1.36% | | 0.60% | | 0.83% | |
| DEC | n.a. | | n.a. | | 0.618% | | 7.206% | | 2.029% | |
| | | | | | | | | | | |
| Villi | average | st.dev. | average | st.dev. | average | st.dev. | average | st.dev. | average | st.dev. |
| Length | 265.96 | 10.59 | 202.67 | 19.92 | 232.85 | 20.88 | 243.73 | 26.28 | 244.00 | 17.41 |
| Thickness | 58.22 | 3.02 | 88.39 | 15.18 | 68.57 | 7.60 | 75.15 | 9.91 | 70.97 | 10.51 |

Surprisingly, mice fed with diets supplemented with polysaccharides with a relative degree of esterification scattering (RDES) of less than 1.7% and/or a degree of esterification clustering (DEC) of more than 0.4% showed a significant repair in the villi length after induced mucositis (indicated by black cells in table 7), suggesting positive impact on immune status.

Moreover, mice fed with diets supplemented with polysaccharides with a relative degree of esterification scattering (RDES) of less than 1.7% and/or a degree of esterification clustering (DEC) of more than 0.4% showed a significant reduction in neutrophil infiltration after induced mucositis (see table 8), suggesting positive impact on immune status

These results suggest that the use esterified polysaccharides with relative degree of esterification scattering (RDES) of less than 1.7% and/or a degree of esterification clustering (DEC) of more than 0.4% can be beneficial for treating, preventing, and/or reducing immune-mediated diseases and inflammatory diseases in mammals.

Table 8: Neutrophil infiltration

| | Control | | Doxorubicin | | Doxorubicin + LP1 | | Doxorubicin + LP3 | | Doxorubicin + LP4 | |
|---|---|---|---|---|---|---|---|---|---|---|
| RDES | n.a. | | n.a. | | 1.36% | | 0.60% | | 0.83% | |
| DEC | n.a. | | n.a. | | 0.618% | | 7.206% | | 2.029% | |
| | | | | | | | | | | |
| | average | st.dev. | average | st.dev. | average | st.dev. | average | st.dev. | average | st.dev. |
| neutrophil infiltration | 8.43 | 3.05 | 71 | 6.24 | 51.57 | 11.22 | 44.33 | 9.56 | 47.8 | 13.85 |

## Claims

1. A method for treating, preventing, and/or reducing immune-mediated diseases and inflammatory diseases in mammals, said method comprising administering to a subject in need thereof an effective amount of at least one esterified polysaccharide, wherein the esterified polysaccharide has a relative degree of esterification scattering (RDES) of less than 1.7%.

2. A method for treating, preventing, and/or reducing immune-mediated diseases and inflammatory diseases in mammals, said method comprising administering to a subject in need thereof an effective amount of at least one esterified polysaccharide, wherein the esterified polysaccharide has a degree of esterification clustering (DEC) of more than 0.4%.

3. A method according to claim 1 or claims 2, wherein immune-mediated disease and inflammatory disease is controlled through activation and/or proliferation of T regulatory cells.

4. A method according to claim 3, wherein T regulatory cells are Foxp3+RORγt+ T regulatory cells.

5. A method according to claim 1 or claim 2, wherein the immune-mediated disease and inflammatory disease is controlled through preventing activation and/or proliferation of Th17 effector cells.

6. A method according to anyone of the preceding claims wherein the polysaccharide is administered at a daily dose of at least 10 mg.

7. A method according to anyone of the preceding claims wherein the subject is a human.

8. The polysaccharide of the invention is also known as pectic polysaccharide or pectin.

9. A composition comprising

   (a) at least one therapeutically inert carrier and
   (b) at least one esterified polysaccharide, wherein the esterified polysaccharide has a relative degree of esterification scattering (RDES) of less than 1.7%.

10. A composition comprising

   (a) at least one therapeutically inert carrier and
   (b) at least one esterified polysaccharide, wherein the esterified polysaccharide has a degree of esterification clustering (DEC) of more than 0.4%.

11. A composition according to claim 9 or 10,wherein the composition comprises 0.5 wt-% to 50 wt-%, based on the total weight of the composition, of the polysaccharide

Europäisches Patentamt
European Patent Office
Office européen des brevets

# EUROPEAN SEARCH REPORT

Application Number
EP 20 16 9672

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/009257 A2 (NUTRITION SCIENCES N V [BE]; ROYAL AGRIFIRM GROUP B V [NL] ET AL.) 19 January 2017 (2017-01-19)<br>* the whole document *<br>----- | 1-11 | INV.<br>A61K31/715<br>A61K31/732<br>A61P29/00<br>A61P35/00 |
| X | LINLIN FAN ET AL: "Preventive effects of pectin with various degrees of esterification on ulcerative colitis in mice",<br>FOOD & FUNCTION,<br>vol. 11, no. 4,<br>24 February 2020 (2020-02-24), pages 2886-2897, XP055727813,<br>GB<br>ISSN: 2042-6496, DOI: 10.1039/C9FO03068A<br>* the whole document *<br>----- | 1-11 | |
| X | CN 107 158 026 A (UNIV JIANGNAN)<br>15 September 2017 (2017-09-15)<br>* abstract *<br>----- | 1-11 | |
| X | US 10 300 085 B2 (DAICEL CORP [JP])<br>28 May 2019 (2019-05-28)<br>* the whole document *<br>----- | 1-11 | TECHNICAL FIELDS SEARCHED (IPC)<br>A61K<br>A61P |
| X | WO 2019/081523 A1 (NUTRILEADS B V [NL])<br>2 May 2019 (2019-05-02)<br>* the whole document *<br>----- | 1-11 | |
| Y | DAAS P J H ET AL: "Characterization of non-esterified galacturonic acid sequences in pectin with endopolygalacturonase",<br>CARBOHYDRATE RESEARCH, PERGAMON, GB,<br>vol. 326, no. 2, 1 June 2000 (2000-06-01), pages 120-129, XP004200122,<br>ISSN: 0008-6215, DOI: 10.1016/S0008-6215(00)00037-9<br>* the whole document *<br>-----<br>-/-- | 1-11 | |

The present search report has been drawn up for all claims

1

| Place of search | Date of completion of the search | Examiner |
|---|---|---|
| Munich | 10 September 2020 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 16 9672

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | PIET J. H. DAAS ET AL: "Nonesterified galacturonic acid sequence homology of pectins", BIOPOLYMERS, vol. 58, no. 1, 1 January 2001 (2001-01-01), pages 1-8, XP055729624, US ISSN: 0006-3525, DOI: 10.1002/1097-0282(200101)58:1<1::AID-BIP10>3.0.CO;2-I * the whole document * ----- | 1-11 | |
| | | | TECHNICAL FIELDS SEARCHED (IPC) |

1

The present search report has been drawn up for all claims

| Place of search | Date of completion of the search | Examiner |
|---|---|---|
| Munich | 10 September 2020 | Houyvet-Landriscina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 20 16 9672

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2020

| Patent document cited in search report | Publication date | Patent family member(s) | Publication date |
|---|---|---|---|
| WO 2017009257 A2 | 19-01-2017 | BR 112018000520 A2 | 18-09-2018 |
| | | CN 107921057 A | 17-04-2018 |
| | | EP 3319616 A2 | 16-05-2018 |
| | | US 2019008890 A1 | 10-01-2019 |
| | | WO 2017009257 A2 | 19-01-2017 |
| CN 107158026 A | 15-09-2017 | NONE | |
| US 10300085 B2 | 28-05-2019 | EP 3130236 A1 | 15-02-2017 |
| | | KR 20160137581 A | 30-11-2016 |
| | | US 2017100426 A1 | 13-04-2017 |
| | | WO 2015146853 A1 | 01-10-2015 |
| WO 2019081523 A1 | 02-05-2019 | AU 2018356377 A1 | 07-05-2020 |
| | | CA 3079933 A1 | 02-05-2019 |
| | | EP 3700357 A1 | 02-09-2020 |
| | | KR 20200078570 A | 01-07-2020 |
| | | US 2020246373 A1 | 06-08-2020 |
| | | WO 2019081523 A1 | 02-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7651855 B
- US 20190358258 A
- US 20160193257 A
- US 20190307795 A
- US 9433652 B


### Non-patent literature cited in the description


- **CHEN et al.** *Int Immunopharmacol.,* 2011, vol. 11 (5), 536-542
- **YANG et al.** *Mucosal Immunol.,* 2016, vol. 9 (2), 444-457
- **LALLÈS et al.** *Proc Nutr Soc.,* 2007, vol. 66 (2), 260-268
- **LALLÈS et al.** *Animal Research,* 2004, vol. 53, 301-316
- **WIJTTEN et al.** *Br J Nutr.,* 2011, vol. 105 (7), 967-981
- **BAUER et al.** *Nutr Res Rev.,* 2006, vol. 19 (1), 63-78
- **TIMBERMONT et al.** *Avian Pathol.,* 2011, vol. 40 (4), 341-347
- **HERMANS et al.** *Vet Rec.,* 2006, vol. 158 (18), 615-622
- **WIJTTEN et al.** *Acta Agriculturae Scandinavica Section A-Animal Science,* 2012, vol. 62 (1), 1-12
- **LUU et al.** *Clin. Transl. Immunology,* 2017, vol. 6 (9), e56
- **CHARBONNIER et al.** *J. Immunology,* 2006, vol. 177 (6), 3806-13
- **MAY.** *Carbohydr. Polymers,* 1990, vol. 12, 79-99
- **ROLIN.** Pectins and their Manipulation. Blackwell Publishing Ltd, 2002, 222-239
- **CHRISTENSEN.** *Pectins. Food Hydrocolloids,* 1986, vol. 3, 205-230
- **KRAVTCHENKO et al.** *Carbohydrate Polymers,* 1992, vol. 19, 115-124
- **VORAGEN et al.** Food polysaccharides and their applications. Marcel Dekker Inc, 1995, 287-339
- **GNANASAMBANDAM ; PROCTOR.** *Food Chemistry,* 2000, vol. 68, 327-332
- **HAAS ; JAGER.** *Journal of Food Science,* 1986, vol. 51 (4), 1087-1088
- **REINTJES et al.** *Journal of food sciences,* 1962, vol. 27, 441-445
- **GRASDALEN et al.** *Carbohydrate Research,* 1988, vol. 184, 183-191
- **CHATJIGAKIS et al.** *Carbohydrate Polymers,* 1998, vol. 37, 395-408
- **LEVIGNE et al.** *Food Hydrocolloids,* 2002, vol. 16 (6), 547-550
- **VORAGEN et al.** *Food Hydrocolloids,* 1986, vol. 1 (1), 65-70
- **HUISMAN et al.** *Food Hydrocolloids,* 2004, vol. 18 (4), 665-668
- **WALTER et al.** *Journal of Food Science,* 1983, vol. 48 (3), 1006-1007
- **JIANG et al.** *Food Chemistry,* 2005, vol. 91, 551-555
- **JIANG et al.** *Journal of Agricultural and Food Chemistry,* 2001, vol. 49, 5584-5588
- **ZHONG et al.** *Carbohydrate Research,* 1998, vol. 308, 1-8
- **ZHONG et al.** *Carbohydrate Polymers,* 1997, vol. 32 (1), 27-32
- Handbook of Food Analytical Chemistry: Water, Proteins, Enzjmes, Lipids, and Carbohydrates. John Wiley & Sons, Inc, 2005
- **RALET et al.** *Biomacromolecules,* 2012, vol. 13, 1615-1624
- **BROXTERMAN et al.** *Carbohydrate Polymers,* 2017, vol. 177, 58-66
- **BLUMENKRANTZ ; ASBOE-HANSEN.** *Anal Biochem,* 1973, vol. 54, 484-489
- Studies on the intra- and intermolecular distributions of substituents in commercial pectins. **STÉPHANIE GUILLOTIN.** PhD thesis. Wageningen University, 2005
- **HUISMAN et al.** *Food Hydrocolloids,* 2004, vol. 18, 665-668
- **THIBAULT ; ROBIN.** Automatisation du dosage des acides uroniques par la méthode de carbazol. Application au cas de matières pectiques. *Ann.Technol.Agric.,* 1975, vol. 24, 99-110
- **ELDERMAN et al.** *Biol Sex Differ.,* 2018, vol. 9, 26